# EUROPEAN PATENT SPECIFICATION

(11) **EP 2 750 684 B1**
(45) Date of publication and mention of the grant of the patent: **17.10.2018**
(21) Application number: 12823450.7
(22) Date of filing: 17.08.2012
(51) Int. Cl.: A61K 38/00, A61K 36/8945, C07K 14/415

(54) **NOVEL BIOACTIVE PROTEIN ISOLATED FROM CHINESE YAM AND USES THEREOF**
NEUES, AUS CHINESISCHER YAMSWURZEL ISOLIERTES BIOAKTIVES PROTEIN UND SEINE VERWENDUNG
NOUVELLE PROTÉINE BIOACTIVE ISOLÉE DE L'IGNAME CHINOISE ET SES UTILISATIONS

(30) Priority: 17.08.2011 US 201161524477 P
(43) Date of publication of application: 09.07.2014
(73) Proprietor: Versitech Limited, Hong Kong (CN)
(72) Inventor: SZE, Chowing, North Point Hong Kong (CN); WONG, Kamlok, Tai Wai New Territories Hong Kong (CN); TONG, Yao, Pokfulam Hong Kong (CN); ZHANG, Yanbo, Hong Kong (CN); CHEUNG, Hopan, Shatin New Territories Hong Kong (CN)
(74) Representative: Epping - Hermann - Fischer
(86) International application number: PCT/CN2012/080322
(87) International publication number: WO 2013/023617

(56) References cited:
- WO-A1-03/040134
- WO-A2-00/77165
- US-B1- 6 238 707
- PARK, M. K. ET AL.: 'Estrogen Activities and the Cellular Effects of Natural Progesterone from Wild Yam Extract in MCF-7 Human Breast Cancer Cells.' AM J CHIN MED vol. 37, no. 1, 2009, pages 159 - 167, XP055142803
- W. H. ET AL.: 'Estrogenic Effect of Yam Ingestion in Healthy Postmenopausal Women.' J AM COLL NUTR vol. 24, no. 4, 01 August 2005, pages 235 - 243, XP008173331

## Description

### CROSS-REFERENCE TO RELATED APPLICATION

This application claims the benefit of U.S. Provisional Application Serial No. 61/524,477, filed August 17, 2011, which is hereby incorporated by reference in its entirety.

### TECHNICAL FIELD

This invention relates to a bioactive protein originated from Chinese yam and uses thereof for increasing serum estrogen and progesterone levels and for treatment of osteoporosis, menopausal syndrome and the accompanying cognitive decline.

### BACKGROUND OF THE INVENTION

Menopause, a normal part of the aging process, is the period during which the level of estrogen and progesterone secreted by the ovaries gradually declines. Approximately 80% of women experience mild or few symptoms. However some women have severe symptoms including: hot flushes, heavy sweating, anxiety, panic, or depression, drying and wrinkling of the skin, vaginal dryness and discomfort, urinary stress incontinence, cystitis, insomnia, irritability and osteoporosis. A report published by the World Health Organization in 1990 estimated that the total population of postmenopausal women in the world was 476 million. By 2030, the predicted population will reach 1.2 billion.

The current treatment to relieve menopausal syndrome is hormone replacement therapy (HRT), which consists of administration of supplementary exogenous estrogen or estrogen plus progestin most frequently using pills, implants under the skin or skin patches. While HRT increases the circulating estrogen level by providing exogenous estrogen to menopausal women, the appropriate dosage and the duration of treatment are difficult to determine due to individual variations in physiological conditions among menopausal women. Consequently, HRT is sometimes ineffective and prone to causing side effects including endometrioma, breast and ovarian cancer, coronary heart disease and stroke.

Osteoporosis results from an increased rate of bone resorption and relatively decreased rate of bone formation, resulting in reduced bone mass and micro-architectural deterioration of the skeleton, and an increased risk of fractures. Bone remodeling increases substantially in the years after menopause and remains elevated in older osteoporosis patients indicating that the loss of ovarian hormones during menopause is one of the major risk factors for osteoporosis. Bone status as such contributes to the increases in age-related skeletal fragility in women. It has been shown that trabecular bone mineral density, trabecular bone volume fraction, trabecular thickness and trabecular number all decrease with age. Also, a lower bone mass is primarily characterized by a smaller plate-to-rod ratio. All these changes weaken bone strength and increase the risk of osteoporotic fracture.

Weight-bearing exercises have an osteogenic effect in postmenopausal women. However, vigorous exercises are needed to engender an osteogenic effect in the elderly. Use of HRT in combination with weight bearing exercises has been shown to have a synergistic effect. This shows that the osteogenic response may be enhanced by the administrated estrogen.

Selective estrogen receptor modulator (SERM) is a remedy in particular for relieving postmenopausal osteoporosis. However, there is no evidence showing that SERM would increase the endogenous estrogen level in menopausal women, and therefore, may not relieve menopausal syndromes other than osteoporosis.

Various studies have shown that proteins or other compounds isolated from different species of yam tuber have anti-oxidative, chitinase and immunomodulatory activities, including activating estrogen receptors.

It is desirable to develop a cost-effective treatment for symptoms of menopausal syndrome in order to alleviate or prevent osteoporosis and/or cognitive decline resulting from low serum levels estrogen and progesterone levels.

WO 03/040134 A1 discloses methods of preparing an estrogenic preparation and isolating an estrogenic compound from a plant, such as an Epimedium plant, and the use of the estrogenic preparation or the isolated estrogenic compound in modulating an estrogen receptor.

The document "Estrogenic Effect of Yam Ingestion in Healthy Postmenopausal Women", J. Am. Coll. Nutr. 2005, vol. 24, no. 4, 235-243 discloses a study which investigates the effects of yam ingestion on lipids, antioxidant status and sex hormones in postmenopausal women.

### BRIEF SUMMARY

The present invention provides a novel polypeptide originated from Chinese yam (*Dioscorea opposita*) tubers. The polypeptide increases circulating estrogen levels, up-regulates the expression of ovarian follicle stimulating hormone receptor (FSHR), elevates ovarian aromatase (ovarian CYP-19), and exhibits anti-osteoporotic activity.

In one embodiment, the present invention provides an isolated or substantially pure polypeptide originated from the rhizomes of *Dioscorea opposita,* wherein the polypeptide has a molecular weight of about 32.5 kDA (as determined by chromatography on a SUPERDEX 75 10/300 GL column) and an N-terminal sequence Gly-Ile-Gly-Lys-Ile-Thr-Thr-Tyr-Trp-Gly-Gln-Tyr-Ser-Asp-Glu-Pro-Ser-Leu-Thr-Glu-Ala (SEQ ID NO:1). In one embodiment, when the peptide is administered to a female subject, serum estrogen and progesterone levels are increased.

It is provided a composition comprising the polypeptide compound according to the first aspect of the present invention or a salt thereof and a pharmaceutically acceptable carrier for use in increasing the level of estradiol, estrogen, and/or progesterone *in vivo* and/or *in vitro,* for increasing the expression levels of aromatase and/or follicle-stimulating hormone receptor (FSHR), and for treatment of menopausal syndrome.

### BRIEF DESCRIPTION OF THE DRAWINGS

The present invention is illustrated by the accompanying drawings of various embodiments and the detailed description given below. The drawings should not be taken to limit the invention to the specific embodiments, but are for explanation and understanding. The detailed description and drawings are merely illustrative of the invention rather than limiting, the scope of the invention being defined by the appended claims and equivalents thereof. The drawings are not to scale. The foregoing aspects and other attendant advantages of the present invention will become more readily appreciated by the detailed description taken in conjunction with the accompanying drawings.
Fig. 1 is a photograph of the Chinese yam *Dioscorea opposita.*
Fig. 2 is a chromatogram showing the purification of an extract of *Dioscorea opposita* on a HiPrep 16/10 DEAE FF column.
Fig. 3 is a chromatogram showing the further purification of a DO protein fraction on a HiPrep 16/10 Phenyl FF (high sub) column.
Fig. 4 is a chromatogram showing the purification of a DO protein fraction on a Superdex 75 10/300 GL column.
Fig. 5 is a photograph of a 15% Native PAGE of DO extract (left) and 15% SDS PAGE (right), showing the protein recovered from the DO extract visualized by silver staining.
Fig. 6A is a chromatogram showing elution of the protein standards blue dextran and ovalbumin on a SUPERDEX 75 10/300 GL column.
Fig. 6B is a chromatogram showing the elution of the protein standards bovine serum albumin and aprotinin on a SUPERDEX 75 10/300 GL column.
Fig. 6C is a chromatogram showing the elution of DO peptide on a Superdex 75 10/300 GL column, in accordance with the present invention.
Fig. 6D is a graph showing a calibration curve from which the molecular weight of DO peptide was determined.
Fig. 7 shows the N-terminal sequence of the isolated DO protein.
Fig. 8 is a graph showing the estrogenic activity of DO protein on granulosa cells, in accordance with the present invention;
Fig. 9 is a graph showing the estrogenic activity of NaOH treated DO peptide in ganulosa cells.
Fig. 10 is a graph showing the estrogenic activity of HCl treated DO peptide on granulosa cells.
Fig 11 is a graph showing the estrogenic activity of heat treated DO peptide on granulosa cells.
Fig. 12A is a graph of serum estrogen (estradiol) levels of Sprague-Dawley rats after a 2-week treatment with DO peptide.
Fig. 12B is a graph of serum estrogen (estradiol) levels of Sprague-Dawley rats after a 4-week treatment period with DO peptide.
Fig. 12C is a graph of serum estrogen (estradiol) levels of Sprague-Dawley rats after a 6-week treatment period with DO peptide.
Fig. 12D is a graph of serum progesterone levels of Sprague-Dawley rats after a 2-week treatment period with DO peptide, in accordance with the present invention.
Fig. 12E is a graph of serum progesterone levels of Sprague-Dawley rats after a 4-week treatment period with DO peptide.
Fig. 12F is a graph of serum progesterone levels of Sprague-Dawley rats after a 6-week treatment period with DO peptide.
Fig. 13 is a graph showing protein expression of ovarian CYP-19 aromatase in granulosa cells.
Fig. 14 is a graph showing protein expression of FSHR in granulosa cells.
Fig. 15 is a graph showing mRNA expression of ovarian CYP-19 aromatase in ovaries from rats after treatment with DO peptide.
Fig. 16 is a graph showing mRNA expression of FSHR in ovaries from rats after treatment with DO.
Fig. 17 is a graph showing the protein expression level of ovarian CYP-19 aromatase in ovaries from rats after treatment with DO peptide.
Fig. 18 is a graph showing the protein expression level of FSHR in ovaries from rats after treatment with DO peptide.
Fig. 19 is a graph showing the effect of DO on proliferation of BT-483 breast cancer cells.
Fig. 20 is a graph showing the effect of DO on proliferation of OVCA-429 ovarian cancer cells.
Fig. 21 is a graph showing the body weight of rats after treatment with DO peptide.
Fig. 22 is a graph showing the apparent trabecular bone mineral density of vertebra L2 of rats after 6-week treatment with DO peptide.
Fig. 23 is a graph showing the bone volume fraction of vertebra L2 of rats after 6-week treatment with DO peptide.
Fig. 24 is a graph of the trabecular number of vertebra L2 of rats after a 6-week treatment period with DO peptide, in accordance with the present invention;
Fig. 25 is a graph of the trabecular thickness of vertebra L2 of rats after a 6-week period of treatment with DO peptide.
Fig. 26 is a graph of the structure model index of vertebra L2 of rats after a 6-week treatment period with DO peptide.
Fig. 27 is a graph of the trabecular separation of vertebra L2 of rats after a 6-week treatment period with DO peptide.
FIG. 28 is a graph of the protein level of brain derived neurotrophic factor (BDNF) protein in the hippocampus of rats after treatment with DO peptide.
FIG. 29 is a graph showing the protein level of brain derived neurotrophic factor (BDNF) protein in the prefrontal cortex of rats after treatment with DO peptide.
FIG. 30 is a graph showing the protein level of TrkB gp145 receptor protein in prefrontal cortex of rats after treatment with DO peptide.
FIG. 31 is a graph showing the results of an assay of mitogenic activity of DO peptide on mouse splenocytes.

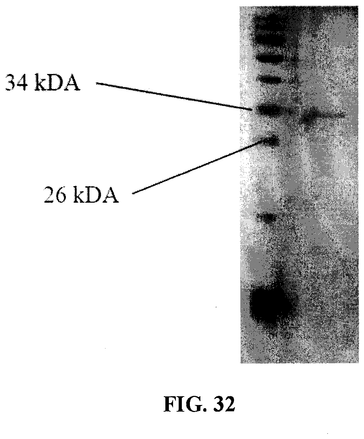

FIG. 32 is a photograph of a 15% SDS PAGE, showing the DO protein isolated by the antibody affinity column visualized by silver staining. The left lane is protein molecular weight standards and right lane showing the eluted DO protein retained by affinity column. The size of DO protein eluted is approximately 30 kDA, when compared to that of molecular weight standards 34kDA and 26 kDA. The apparent molecular weight of the DO peptide is consistent with that obtained using conventional column purification techniques.
FIG. 33 shows the estrogenic activity of DO protein on ovarian granulosa cells. The DO protein is isolated from *Dioscorea opposita* using antibody affinity column. The results are expressed as means ± SEM, n=3. ** *p* < *0.01* compared with control group by un-paired *t-test.*
FIG. 34 shows the effect of the DO protein on expression of aromatase in mammary gland.

### BRIEF DESCRIPTION OF THE SEQUENCES

**SEQ ID NO:1** is the N-terminal sequence of a novel protein (DO) purified from Chinese yam tuber *Dioscorea opposita.*
**SEQ ID NO:2** is a primer sequence useful according to the present invention.
**SEQ ID NO:3** is a primer sequence useful according to the present invention.
**SEQ ID NO:4** is a primer sequence useful according to the present invention.
**SEQ ID NO:5** is a primer sequence useful according to the present invention

### DETAILED DESCRIPTION

Throughout this specification, like numbers refer to like elements.

The present invention is directed to a novel protein (the DO protein or peptide) isolated from the Chinese yam tuber *Dioscorea opposita.* In one embodiment, the purified DO peptide elevates circulating estrogen levels by up-regulating the expression of ovarian follicle stimulating hormone receptor (FSHR) and ovarian aromatase (ovarian CYP-19). In another embodiment, the DO peptide reduces or prevents the decalcification of bone and osteoporosis. In another embodiment the DO peptide increases the level of brain derived neurotrophic factor (BDNF) in the hippocampus and increases the levels of BDNF and TrkB gp 145 receptor protein in the prefrontal cortex.

### Proteins

In one embodiment, the present invention provides an isolated or substantially pure polypeptide having an apparent molecular weight of about 32.5 kDa, wherein the first twenty-one consecutive amino acids at the N-terminal of the polypeptide consists of SEQ ID NO: 1. In one embodiment, the polypeptide originates from *Dioscorea* sp. In one specific embodiment, the polypeptide originates from *Dioscorea opposita.*

In one embodiment, the polypeptide of the present invention has characteristics of the DO peptide as shown in Table 1.

In one embodiment, the polypeptide of the present invention increases the level of estradiol, estrogen, and/or progesterone *in vivo* and/or *in vitro.* In one specific embodiment, the polypeptide of the present invention increases the level of estradiol, estrogen, and/or progesterone in ovarian tissue and/or the blood (e.g. serum, plasma, whole blood). In one embodiment, the polypeptide of the present invention increases the expression of aromatase and/or follicle-stimulating hormone receptor (FSHR). In one specific embodiment, the polypeptide of the present invention increases the ratio of expression level of aromatase:GAPDH and/or FSHR:GAPDH.

In one embodiment, the polypeptide of the present invention has higher activity (such as increasing the level of estradiol, estrogen, and/or progesterone) after incubation at pH about 1 than after incubation at pH 0 or at pH 2. In one embodiment, the polypeptide of the present invention has higher activity (such as increasing the level of estradiol, estrogen, and/or progesterone after incubation at 80° C than after incubation at 60° C or at 100 °C.

In one embodiment, the polypeptide of the present invention has activity (such increasing the level of estradiol, estrogen, and/or progesterone) after incubation at pH 0. In one embodiment, the polypeptide of the present invention has activity (such as increasing the level of estradiol, estrogen, and/or progesterone) after incubation at 100°C.

In one embodiment, the polypeptide of the present invention does not increase the proliferation of breast cancer cells. In one embodiment, the polypeptide of the present invention does not increase the body weight of a subject. In one embodiment, the polypeptide of the present invention increases bone mineral density, bone volume fraction, trabecular number, and/or trabecular thickness in a subject (including a subject with low levels of estrogen and/or progesterone). In one embodiment, the polypeptide of the present invention decreases structure model index and/or trabecular separation in a subject (including a subject with low levels of estrogen and/or progesterone).

In one embodiment, the polypeptide of the present invention increases the levels of brain derived neurotrophic factor (BDNF) and/or TrkB gp145 receptor in the brain of a subject (including a subject with low levels of estrogen and/or progesterone).

In one embodiment, the present invention provides an isolated or substantially pure polypeptide having an apparent molecular weight of about 32.5 kDa, wherein the N-terminal sequence of the polypeptide consists of SEQ ID NO:1, wherein the polypeptide increases the level of estradiol, estrogen, and/or progesterone *in vivo* and/or *in vitro.*

In one embodiment, the polypeptide of the present invention has an apparent molecular weight of about 32.5 kDa.

In one embodiment, the present invention provides an isolated or substantially pure polypeptide, wherein the polypeptide has an apparent molecular weight of about 32.5 kDa and the first twenty-one consecutive amino acids of the N-terminal of the peptide consists of SEQ ID NO:1, wherein the polypeptide increases the level of estradiol, estrogen, and/or progesterone *in vivo* and/or *in vitro.*

In one embodiment, the present invention provides proteins and fusion constructs comprising a polypeptide of the present invention.

The polypeptides of the present invention can be naturally-occurring or can be recombinantly-produced .

In a reference example, an isolated aqueous extract from a *Dioscorea species* (e.g.. *Dioscorea opposita*), wherein the extract comprises a polypeptide of the present invention, is disclosed.

In certain embodiments, the present invention pertains to isolated or substantially pure proteins and polypeptides. The term "substantially pure," as used herein, refers to more than 99% pure.

As used herein, "isolated" refers to extracts and compounds (e.g., proteins and polypeptides) that have been removed from any environment in which they may exist in nature. For example, an isolated extract or compound would not refer to the compound or extract as it exists in plants from which the compound can be isolated. In preferred embodiments, the compounds and extracts of the present invention are at least 75% pure, preferably at least 90% pure, more preferably are more than 95% pure and most preferably are more than 99% pure (substantially pure).

### Treatment of Menopausal Syndrome and Other Diseases

According to a modification a composition is provided, which comprises the polypeptide compound according to the first aspect of the present invention or a salt thereof and a pharmaceutically acceptable carrier for use in increasing the level of estradiol, estrogen, and/or progesterone *in vivo* and/or *in vitro,* for increasing the expression levels of aromatase and/or follicle-stimulating hormone receptor (FSHR), and for treatment of menopausal syndrome.

According to modification a composition is provided, which comprises the polypeptide compound according to the first aspect of the present invention or a salt thereof and a pharmaceutically acceptable carrier for use in increasing the level of a female reproductive hormone (such as estradiol, estrogen, and/or progesterone) in a cell, comprising administering to a cell an effective amount of a composition comprising a polypeptide compound of the invention or a salt thereof.

In one embodiment, the cell is in a subject in need of an increase in the level of a female reproductive hormone (e.g., estradiol, estrogen, and/or progesterone). In one embodiment, the cell is an ovarian cell. In one embodiment, the subject has a low serum level of a female reproductive hormone (e.g., estradiol, estrogen, and/or progesterone), when compared to the level of a normal female population with normal reproductive function. In one embodiment, the subject is a female human subject, and the normal female population consists of females at the age of 14-40 with normal reproductive function. In one embodiment, the subject is a female at menopause, perimenopause, or postmenopause period.

In one embodiment, the present invention does not increase the proliferation of breast cancer cells. In one embodiment, the present invention does not increase the body weight of a subject.

In one embodiment, the present invention can be used to treat osteoporosis of a subject. In one embodiment, the subject is a female having estradiol, estrogen, and/or progesterone levels lower than that a normal female population with reproductive function. In one embodiment, the present invention can be used to improve cognitive function of a subject. In one embodiment, the subject is a female having estradiol, estrogen, and/or progesterone levels lower than that a normal female population with normal reproductive function. In one embodiment, the present invention can be used to increase the levels of brain derived neurotrophic factor (BDNF) and/or TrkB gp145 receptor in the brain of a subject.

The term "subject" or "patient," as used herein, describes an organism, including mammals such as primates, to which treatment with the compositions according to the present invention can be provided. Organisms that can benefit from the disclosed methods of treatment include, but are not limited to, apes, chimpanzees, orangutans, humans, monkeys; domesticated animals such as dogs, cats, horses, cattle, pigs, sheep, goats, chickens; and other animals such as mice, rats, guinea pigs, and hamsters. In one embodiment, the subject or patient is a female.

The term "treatment" or any grammatical variation thereof (e.g., treat, treating, and treatment *etc*.), as used herein, includes but is not limited to, ameliorating or alleviating a symptom of a disease or condition, reducing, suppressing, inhibiting, lessening, or affecting the progression, severity, and/or scope of a condition.

The term "effective amount," as used herein, refers to an amount that is capable of treating or ameliorating a disease or condition or otherwise capable of producing an intended therapeutic effect.

As a reference example, a method of treating osteoporosis comprising administering an aqueous extract of a *Dioscorea opposita* plant rhizome to a patient, and thereby stimulating estrogen and progesterone secretion is provided.

As a reference example,a method of treating menopausal syndrome by administering an aqueous extract of a *Dioscorea opposita* plant rhizome to a patient and stimulating estrogen and progesterone secretion is provided.

As a reference example,a method of treating osteoporosis in a patient by administering an aqueous extract of a *Dioscorea opposita* plant rhizome to the patient and up-regulating follicle stimulating hormone receptor and ovarian aromatase CYP-19 is provided.

As a reference example,a method of treating menopausal syndrome by administering an aqueous extract of a *Dioscorea opposita* plant rhizome to the patient and stimulating estrogen and progesterone secretion in response to the treatment is provided.

As a reference example, a method of treating reduced cognitive function in a patient having low serum estrogen and progesterone levels by administering an aqueous extract of a *Dioscorea opposita* plant rhizome to the patient and thereby raising serum estrogen and progesterone levels and improving cognitive function in response to the raised serum estrogen and progesterone levels is provided.

As a reference example,a method of improving cognitive function in a patient by administering to the patient an aqueous extract of a *Dioscorea opposita* plant rhizome and elevating brain-derived neurotrophic factor expression in the hippocampus and cortex of the patient's brain in response to the extract is provided.

As a reference example,a method of improving cognitive function in a patient by administering to the patient an aqueous extract of a *Dioscorea opposita* plant rhizome and elevating TRKB receptor in the prefrontal cortex of the patient's brain in response to the extract is provided.

### Protein Extraction form Dioscorea opposita

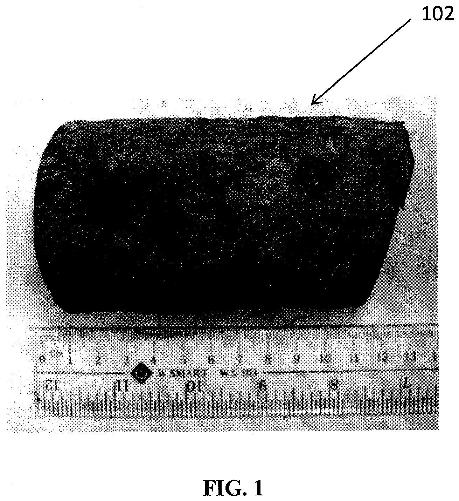

Rhizomes 102 of *Dioscorea opposita* (FIG. 1) were peeled and homogenized in an aqueous extraction buffer (5% acetic acid + 0.1% β-mercaptoethanol) in a ratio of 1:2 (w/v) for 3 hours at 4°C. The homogenate was subjected to centrifugation at 17,700g for 30 min at 4°C. The supernatant was collected and ammonium sulfate was added to 80% of saturation. The mixture was stirred at 4°C overnight and subjected to centrifugation at 17,700g for 1 hr at 4°C. The supernatant was collected and ammonium sulfate was added to 80% of saturation. The mixture was stirred at 4°C overnight and subjected to centrifugation at 17,700g for 1 hr at 4°C. The precipitate (protein extract) was retained and resuspended in purified water. The protein extract mixture was dialyzed against doubly distilled H₂O overnight and then subjected to ultra-centrifugation at 40,000g for 2 hr at 4°C. The supernatant was collected and subjected to fast protein liquid chromatography (FPLC).

### Column purification

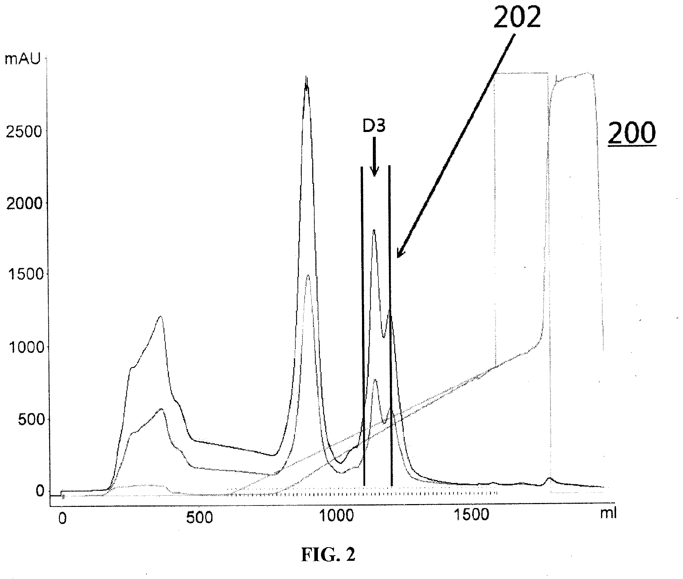

A sample of the above extract was adjusted to a final concentration of 100 mM Tris (pH 8.0) and applied to a HiPrep 16/10 DEAE FF column (GE Healthcare, Inc., Princeton, NJ) and chromatographed by FPLC. The elution buffer consisted of buffer A: 100mM Tris (pH 8.0), and buffer B: 1M NaCl containing 100 mM Tris (pH 8.0). A gradient 0 - 45% Buffer B was used. The elution profile is shown as chromatogram 200 in FIG. 2. Fraction D3 (202) was collected and dialyzed against double distilled H₂O overnight.

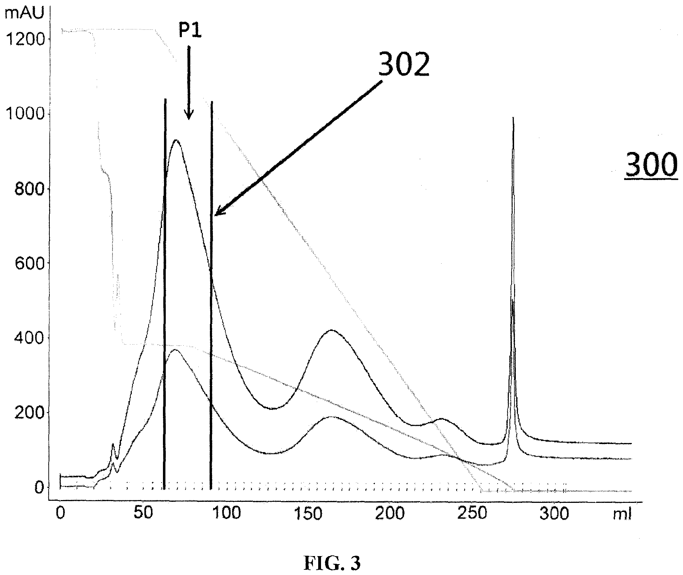

Fraction D3 (202) was then adjusted to 50% Buffer B and applied to a HiPrep 16/10 Phenyl FF (high sub) column (GE Healthcare, Inc., Princeton, NJ), and chromatographed by FPLC. An elution buffer consisting of milli-Q H₂O, and 10 mM sodium phosphate buffer (pH 7.0) containing 1M (NH₄)₂SO₄, (buffer B) was used. The column was eluted with a gradient of 30 - 0% buffer B. The elution profile 300 is shown in FIG. 3. Fraction P1 (302) was collected and dialyzed against double distilled H₂O overnight and then lyophilized to obtain a powdered residue.

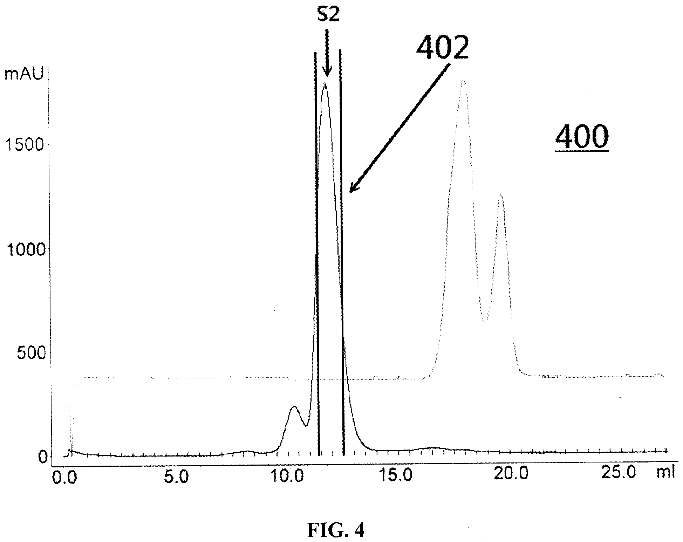

The lyophilized powder from 302 was dissolved in 50 mM sodium phosphate buffer (pH 7.2) containing 150 mM NaCl, applied to a Superdex 75 10/300 GL column (GE Healthcare, Inc., Princeton, NJ) and chromatographed by FPLC. The column was eluted with a 50 mM sodium phosphate buffer (pH 7.2) at a flow rate of 0.6 ml/min. The chromatogram 400 is shown in FIG. 4. Fraction S2 (402) was collected and dialyzed against double distilled H₂O overnight and lyophilized to dryness. The lyophilized residue was recovered and stored at -20°C. This powdered residue is referred to as DO peptide, and is furthered characterized to evaluate its physical, chemical and biological properties.

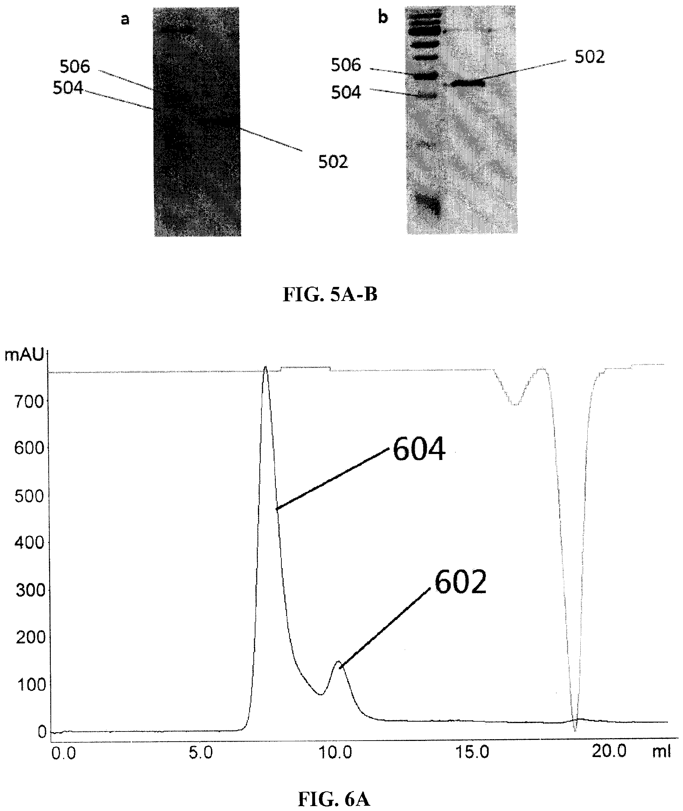

A portion of the powdered residue DO was subjected to polyacrylamide gel electrophoresis (PAGE) on a 15% native PAGE system, and on a 15% sodium dodecyl-sulfate (SDS) PAGE system, as shown in FIG. 5A and 5B, respectively. DO peptide was found to migrate as a single band 502 in both systems, indicating that the DO residue is a relatively pure peptide. The migration of the DO peptide fraction was compared to that of molecular weight standards 504, having a molecular weight of 26 kDA and 506 having a molecular weight of 34 kDA. The molecular weight of DO peptide was found to be approximately 30 kDA as determined from its migration on SDS PAGE.

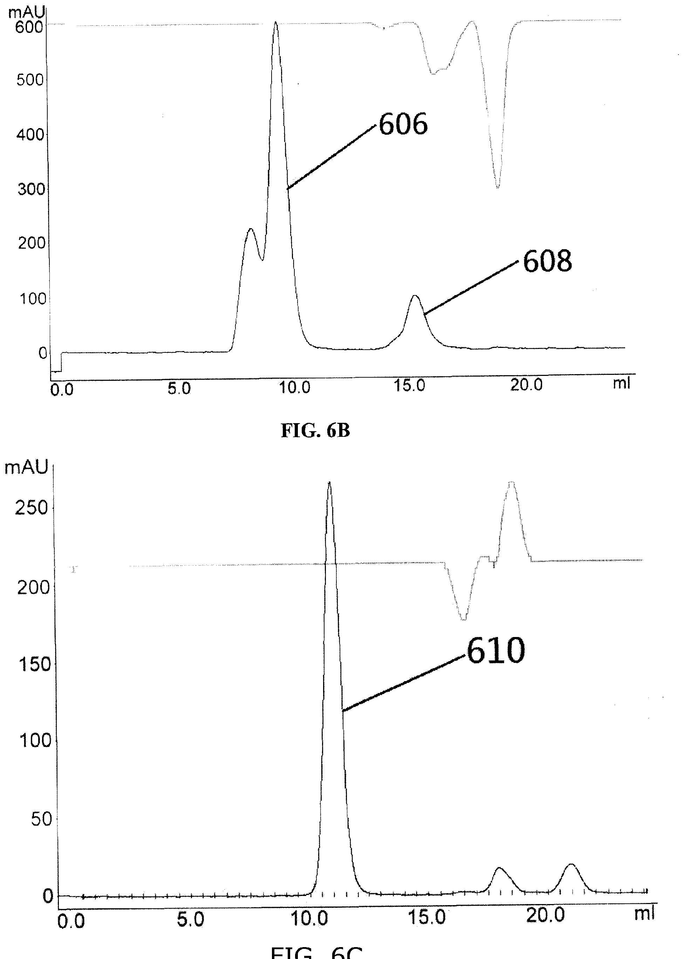

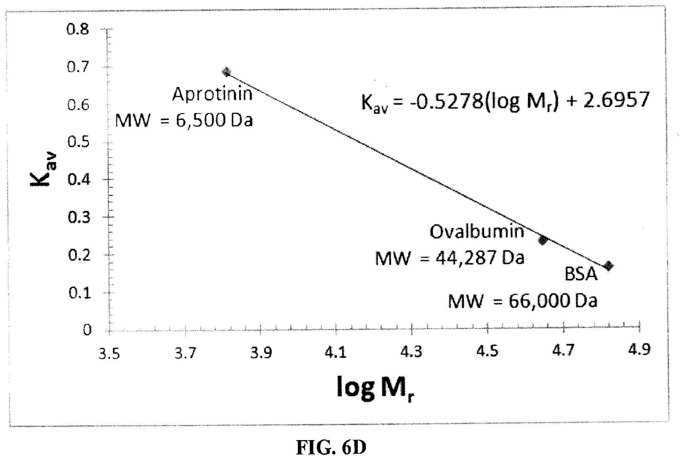

The native size of DO peptide (fraction S2) was determined by its retention time on a Superdex 75 10/300 column using Aprotinin (6500 Da), ovalbumin (44287 Da), and bovine serum albumin (66000 Da) as calibration size standards. The chromatogram showing the elution time of ovalbumin 602 as well as the column front elution volume, indicated by blue dextran 604, is shown in FIG. 6A. The chromatogram showing the elution time of bovine serum albumin, 606 and aprotinin 608 is shown in FIG. 6B. A standard curve, shown in FIG. 6D, was constructed by plotting the elution volumes against the molecular weights of the three standards. Finally, the purified fraction S2 (DO peptide) 610, was chromatographed and the chromatogram is shown in FIG. 6C. The size calculated for the DO peptide from standard curve was 32.5 kDa.

A summary of the molecular weight determination of the DO peptide as determined from its chromatographic elution volume is presented in Table 1:

**Table 1 Calculation of molecular weight of DO:**

| | | | | |
|---|---|---|---|---|
| Superdex 75 10/300 GL | | | | |
| Bed volume (ml) | 18.8 | | | |
| Void volume (ml) | 7.52 | | | |

| | Retention volume (ml) | Kav | Molecular weight (MW) | log MW |
|---|---|---|---|---|
| Bovine serum albumin | 9.35 | 0.162234043 | 66000 | 4.819544 |
| Ovalbumin | 10.121 | 0.230585106 | 44287 | 4.646276 |
| Aprotinin | 15.25 | 0.685283688 | 6500 | 3.812913 |
| DO | 11.06 | 0.313829787 | 32536.47007 | 4.51237 |

The molecular weight of the DO peptide was calculated to be approximately 32.5kDa. A summary of the purification of the DO peptide is presented in Table 2. DO peptide was found to be approximately 0.017% of the total protein in the DO rhizome.

**Table 2**

| | Total protein (mg) / 100g *Dioscorea opposita* | Recovery rate (%) |
|---|---|---|
| Homogenate | 310.788 | 100 |
| Supernatant obtained after ultracentrifugation | 14.250 | 4.585 |
| D3 | 5.071 | 1.632 |
| P1 | 0.570 | 0.183 |
| S2 (DO) | 0.053 | 0.017 |

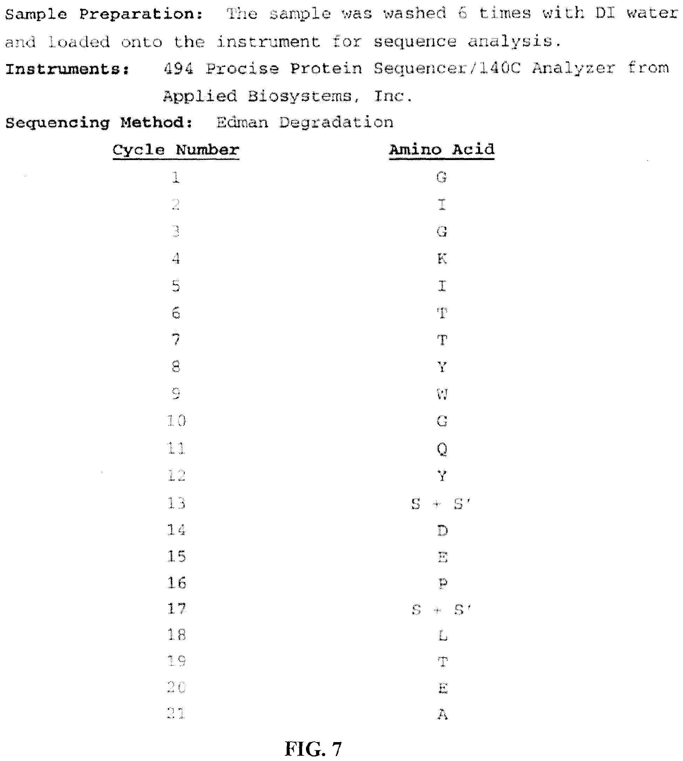

N-terminal sequence analysis of the isolated DO peptide was carried out_on an Model 494 Precise Protein Sequencer and a 140 Analyser (Applied Biosystems, Inc., Carlsbad, CA) using the Edman Degradation process. The results of the sequencing analysis are shown in FIG. 7. N-terminal sequence 700 was found to be:
Gly-Ile-Gly-Lys-Ile-Thr-Thr-Tyr-Trp-Gly-Gln-Tyr-Ser-Asp-Glu-Pro-Ser-Leu-Thr-Glu-Ala (SEQ ID NO:1).

### Cell Culture

Female Sprague-Dawley rats (SD-rats), 21 to 23 day-old, were primed with 80 IU of pregnant mare serum gonadotropin (PMSG) (Sigma) for 48 hours to stimulate follicular development. The rats were then sacrificed and ovaries were dissected. The ovarian follicles were punctured with a 25-gauge needle and granulosa cells were extracted. The granulosa cells were then cultured for 2 hours in serum-free DME/F12 1:1 medium supplemented with penicillin-streptomycin and 1% BSA at 37°C in an atmosphere of 5% CO₂. The DO peptide was then added to the granulosa cells and incubated for 12 hours. The cell culture medium was collected for measurement of estrogen concentration and the cells were harvested for RNA and protein isolation.

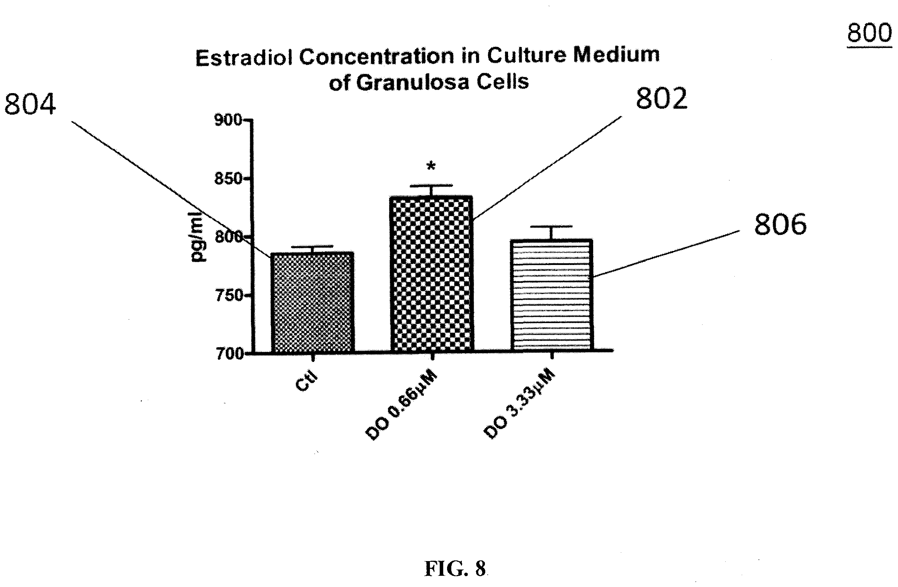

As shown in graph 800, FIG. 8, after a 12 hour incubation period, the average estradiol level in the culture medium of granulosa cells treated with DO peptide at 0.66µM (802) and 3.33µM (806) were 832.2 ± 10.12 pg/ml and 794.4 ± 12.05 pg/ml respectively. There was a significant increase in estradiol level in 0.66µM DO-treated group 802 (p = 0.016, un-paired t-*test*) compared with control 804 (785.5 ± 5.766 pg/ml).

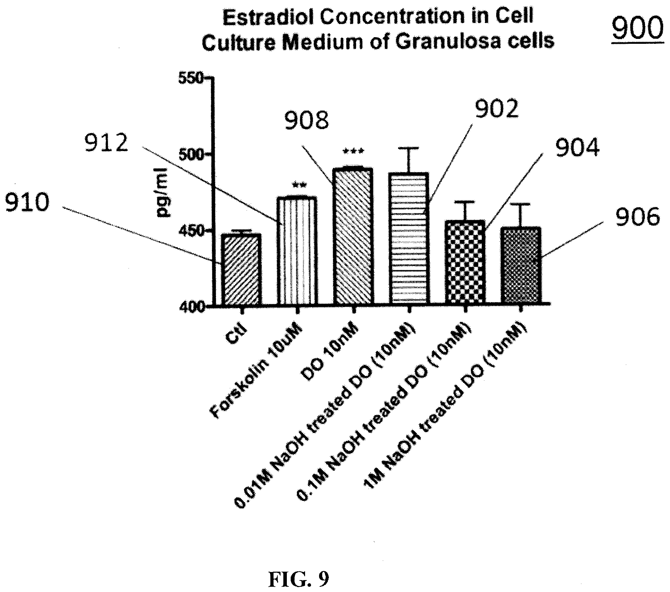

In order to determine the effect of pH on DO peptide, DO peptide was incubated with NaOH at a concentration of 0.01M, 0.1M and 1M respectively, at 4°C for 30 minutes (DO : NaOH = 1:1 by volume ratio). The mixtures were then neutralized with HCl with concentration of 0.01M, 0.1M and 1M, respectively (NaOH : HCl = 1:1 by volume ratio). The treated DO was then added to the granulosa cells and incubated for 12 hours. The cell culture medium was collected for measurement of estradiol concentration. The results showing the effect of basic pH on the activity of DO peptide are presented in graph 900, shown in FIG. 9.

The average estradiol level in the culture medium of granulosa cells treated with 10nM DO, pretreated with 0.01M (902), 0.1M (904) and 1M NaOH (906) were 486.1 ± 16.86 pg/ml, 454.6 ± 12.91 pg/ml and 449.7 ± 15.97 pg/ml respectively. There was no significant difference in estradiol concentration, compared to control group 910, having no added DO peptide. Estradiol concentration for control group 910 (no added DO peptide) was found to be (446.8 ± 3.04 pg/ml). A positive control group 908 comprising DO peptide not exposed to NaOH, showed increased release of estradiol. When exposed to forskolin (912), as a viability test for the cells, the granulosa cells increased estradiol release (912) as expected.

DO was incubated with HCl at a concentration of 0.01M, 0.1M and 1M respectively, at 4°C for 30 minutes (DO peptide : HCl = 1:1 by volume ratio). The mixtures were then neutralized with NaOH with concentration of 0.01M, 0.1M and 1M, respectively (NaOH : HCl = 1:1 by volume ratio). The treated DO was then added to the granulosa cells and incubated for 12 hours. The cell culture medium was collected for measurement of estradiol concentration.

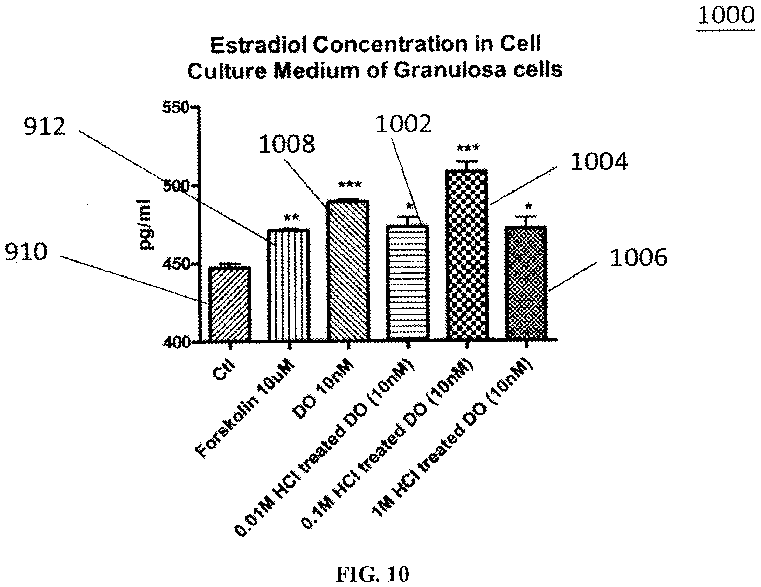

Ovarian granulosa cells incubated with DO peptide that had been exposed to HCl were compared with cells exposed to DO peptide having had no exposure to acid. The results are presented in graph 1000 of FIG. 10. The average estradiol level in culture medium of granulosa cells treated with 10nM DO peptide pretreated with 0.01M (1002), 0.1M (1004) and 1M (1006) HCl were 473.1 ± 5.92 pg/ml, 508.1 ± 6.33 pg/ml and 471.8 ± 6.1 pg/ml respectively. There was a significant increase in estradiol level in all HCl treated groups (p = 0.017, 0.0009 and 0.029 respectively, un-paired *t-test)* compared with control 910 (446.8 ± 3.04 pg/ml). Estradiol release from the acid treated groups was similar to that of the DO peptide (no acid exposure) treated group (1008).

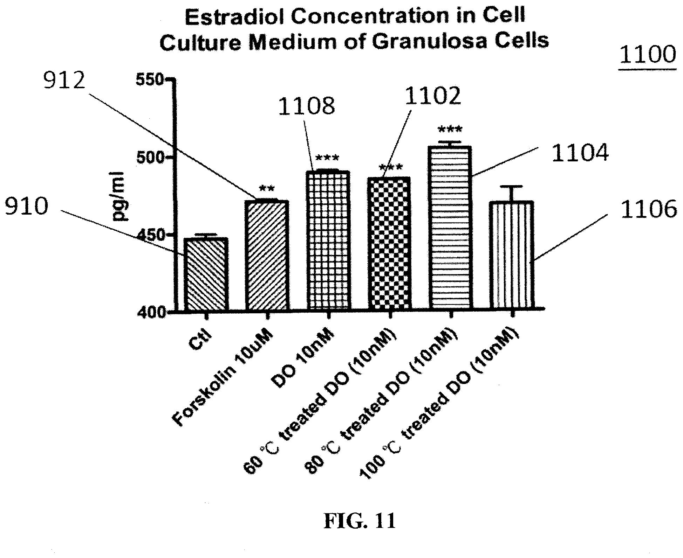

In another embodiment, DO was incubated at 60°C, 80°C and 100°C for 30 minutes. The treated DO was then added to the granulosa cells and incubated for 12 hours treatment. The cell culture medium was collected for measurement of estrogen concentration. The results are shown in graph 1100 shown in FIG. 11.

The average estradiol level in a culture medium of granulosa cells treated with 10nM DO incubated in 60°C, 80°C and 100°C was 484.9 ± 0.46 pg/ml, 504.9 ± 3.38 pg/ml and 468.5 ± 10.62 pg/ml respectively. There was a significant increase in estradiol level in treatment groups with DO incubated in 60°C (1102) and 80°C (1104) (*p* = 0.0002 un-paired *t-test*) compared with control 910 (446.8 ± 3.04 pg/ml). Estradiol released from all treated groups were similar to estradiol release by group 1108, which was treated with DO peptide not exposed to heat.

In one embodiment, the estrogenic activity of DO could be maintained under acidic condition with highest activity after treatment with 0.1M HCl (pH 1). After treated with high temperature (60°C and 80°C), the activity of DO was also stable. However, the activity diminished after treatment with NaOH with a dose dependent manner.

### Animals

Female Sprague-Dawley rats (SD-rats), 18 months old, with low serum estrogen levels were used as an animal model of aging. Female SD-rats, aged 8 months, were purchased from the Laboratory Animal Unit, The University of Hong Kong. The animals were housed in an air-conditioned room at an ambient temperature of 24 °C and 50-65% relative humidity with automatic 12 h light:dark cycles. The experiment had been approved by the Committee on the Use of Live Animals in Teaching and Research (CULATR) of Li Ka Shing Faculty of Medicine, the University of Hong Kong.

### Drug administration, sera and organ collection

Female Sprague-Dawley rats (SD-rats) were randomly divided into five groups: Group 1: rats aged 18 months (control group) were treated by intraperitoneal injection with phosphate buffered saline (PBS); groups 2, 3 and 4: DO were dosed daily by intraperitoneal injection with DO peptide at three different dosages: 2.5, 5 and 10 mg/kg, respectively; Group 5 was a drug group (positive control group), and received oral administration of Premarin (12.4mg/kg). Each rat was treated daily for 6 weeks. Their sera were collected from the tail vein once every two weeks to obtain blood samples for measuring the serum level of estrogen and progesterone. Their ovaries and the 1^{st} to 6^{th} lumbar vertebrae were collected at the end of the experiment and stored at -80°C for further analysis.

### Detection of Serum Hormone Levels

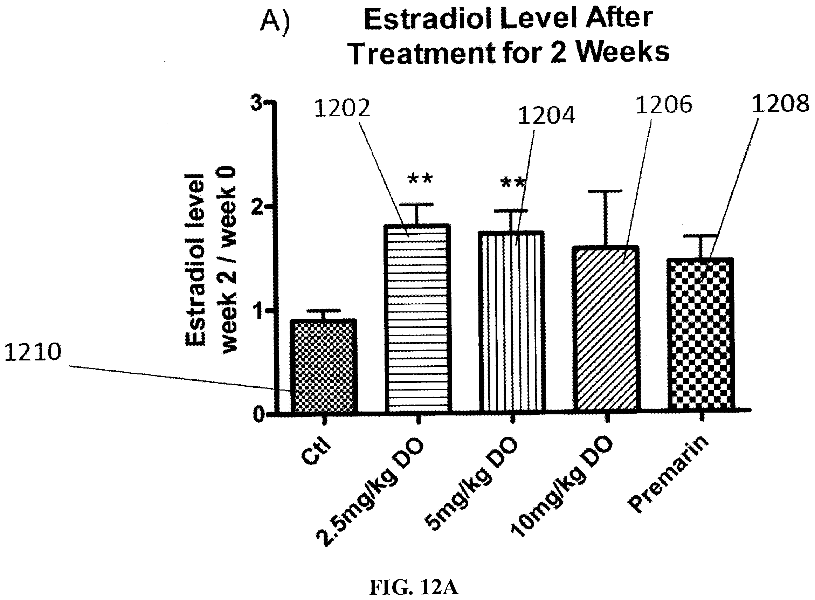

Serum estradiol and progesterone levels were measured by means of an electro-chemiluminescence immunoassay (Elecsys 2010; Roche Diagnostics), following the manufacturer's instruction. As shown in FIG. 12A, after treatment with 2.5 mg/kg (1202), 5 mg/kg (1204), and 10 mg/kg (1206) (daily intraperitoneal injection) of DO peptide or Premarin (positive control, 1208, 12.4mg/kg daily oral administration) for 2 weeks, the serum estradiol levels of the treated rats were respectively 180.1 ± 20.73, 172.4 ± 21.54, 157.7 ± 54.39 and 144.9 ± 22.92 percent (Mean ± SEM, n=6) of the pre-treatment value. The serum estradiol level of negative control group 1210 (PBS) fell to 89.54 ± 9.716 percent of the pre-treatment value. There was a significant increase in the fold change of serum estradiol level in 1202 (2.5 mg/kg) and 1204 (5 mg/kg) DO peptide treated groups (p = *0.0027* and *0.0057* respectively, un-paired *t-test)* compared with control group 1210 (Fig. 12A).

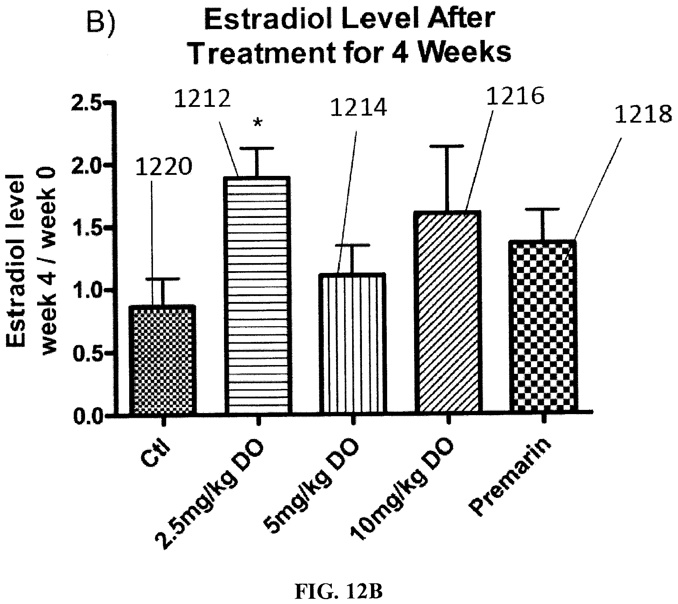

After treatment with 2.5 mg/kg (1212), 5 mg/kg (1214), 10 mg/kg (1216) daily by intraperitoneal injection of DO peptide or Premarin (1218) (positive control 12.4mg/kg daily oral administration) for 4 weeks, the serum estradiol levels of various groups of the Sprague-Dawley rats were respectively 188.8 ± 23.54, 110.6 ± 23.78, 159.9 ± 53.02 and 135.8 ± 26.2 percent (Mean ± SEM, n=6) of the pre-treatment value (FIG. 12B). The serum estradiol level of negative control group 1220 (PBS) fell to 86.24 ± 22.29 percent of the pre-treatment value. There was significant increase in the fold change of serum estradiol level in 1212 (2.5 mg/kg DO treated group) (p = *0.0101,* un-paired *t-test*) compared with control group 1220 (Fig. 12B)

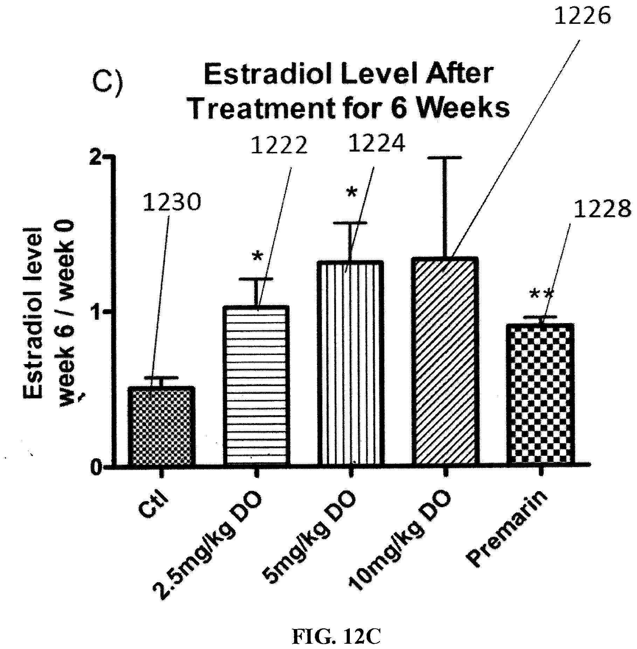

After treatment with 2.5 mg/kg (1222), 5 mg/kg (1224), and 10 mg/kg (1226) by intraperitoneal injection of DO or Premarin (1228) (positive control 12.4mg/kg daily by oral administration) for 6 weeks, the serum estradiol levels of various groups of the Sprague-Dawley rats were respectively 102.4 ± 18.26, 133.1 ± 25.62, 133 ± 65.24 and 89.82 ± 5.24 percent (Mean ± SEM, n=6) of the pre-treatment value, as shown in FIG. 12C. The serum estradiol level of negative control group 1230 (PBS) fell to 50.64 ± 6.88 percent of the pre-treatment value. There was significant increase in the serum estradiol level in both 1222 (2.5 mg/kg) and 1224 (5 mg/kg) DO peptide treated groups and 1228, the Premarin treated group, (p = *0.0242, 0.0126 and 0.0011* respectively, un-paired *t-test)* compared with negative control group 1230, shown in FIG. 12C.

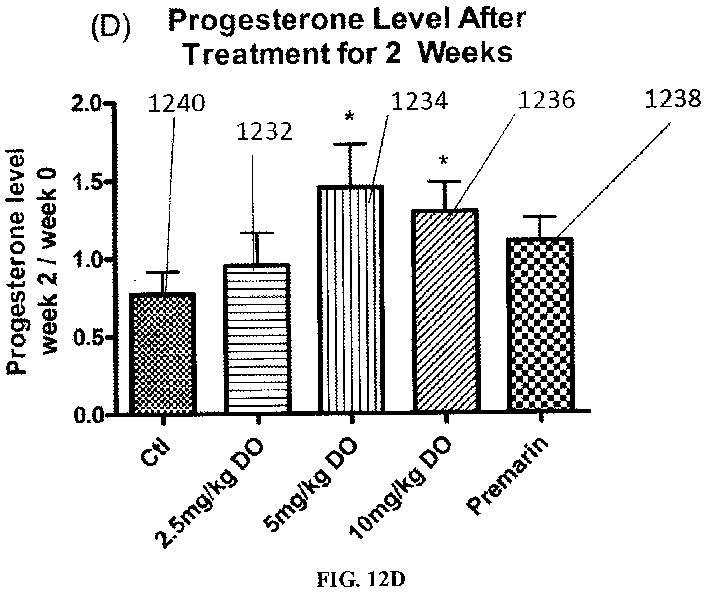

After treatment by daily intraperitoneal injection of DO peptide or Premarin 1238 (positive control 12.4 mg/kg daily oral administration) for 2 weeks, the serum progesterone levels of various groups of the Sprague-Dawley rats, shown in FIG. 12D, were respectively 95.31 ± 21.02 % (1232), 145.3 ± 27.26 % (1234), 129.4 ± 18.76 % (1236) and 110.5 ± 14.8 % (1238) (Mean ± SEM, n ≥ 5) of the pre-treatment value for animals given 2.5 mg/kg, 5 mg/kg, 10 mg/kg of DO peptide and Premarin. The serum progesterone level of negative control group 1240 (PBS) fell to 77.21 ± 14.33 % of the pre-treatment value. There was significant increase in the fold change of serum progesterone level in 5 mg/kg and 10 mg/kg DO peptide treated groups (p = *0.039* and *0.047* respectively, un-paired *t-test)* compared with control group 1240.

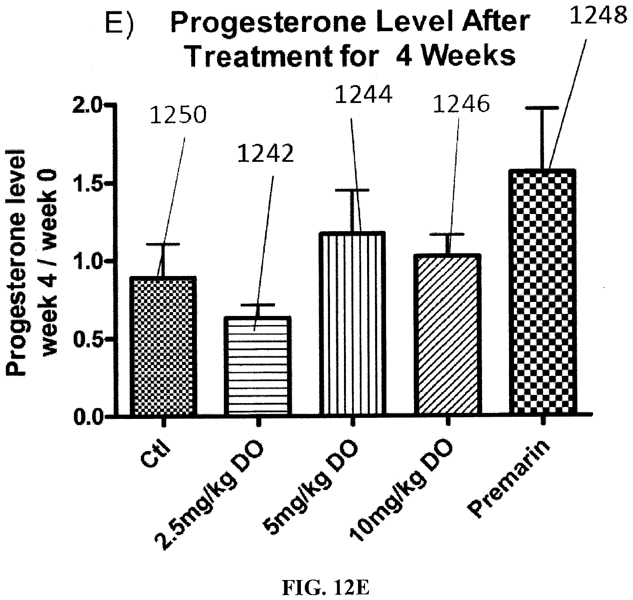

Following daily treatment with 2.5, 5, and 10 mg/kg of DO peptide for 4 weeks, the serum progesterone levels of the various groups of rats were respectively 63.12 ± 8.509 % (1242), 117.0 ± 28.20 % (1244), 102.5 ± 13.50 % (1246) (Mean ± SEM, n ≥ 5) of the pre-treatment value (FIG. 12E). A dose of 12.4 mg/kg daily of Premarin elevated serum progesterone to 156.6 ± 40.07 % (1248) (Mean ± SEM, n ≥ 5) of the pre-treatment value. The serum progesterone level of negative control group 1250 (PBS) fell to 89.16 ± 21.59 % of the pre-treatment value.

After treatment with various doses (2.5 mg/kg, 5 mg/kg, 10 mg/kg daily intraperitoneal injection) of DO and Premarin (positive control 12.4 mg/kg daily oral administration) for 6 weeks, the serum progesterone levels of various groups of the Sprague-Dawley rats were respectively 400.4 ± 182.6 % (1252), 249.9 ± 41.0 % (1254), 249.8 ± 80.89 % (1256) and 194.9 ± 41.96 % (1258) (Mean ± SEM, n ≥ 5) of the pre-treatment value. The serum progesterone level of negative control group 1260 (PBS) rose to 126.4 ± 26.45 % of the pre-treatment value. There was significant increase in the fold change of serum progesterone level in the 5 mg/kg DO peptide treated group 1254 (p = *0.022,* un-paired *t-test*) compared with control group 1260.

### Protein extraction and Western blotting analysis

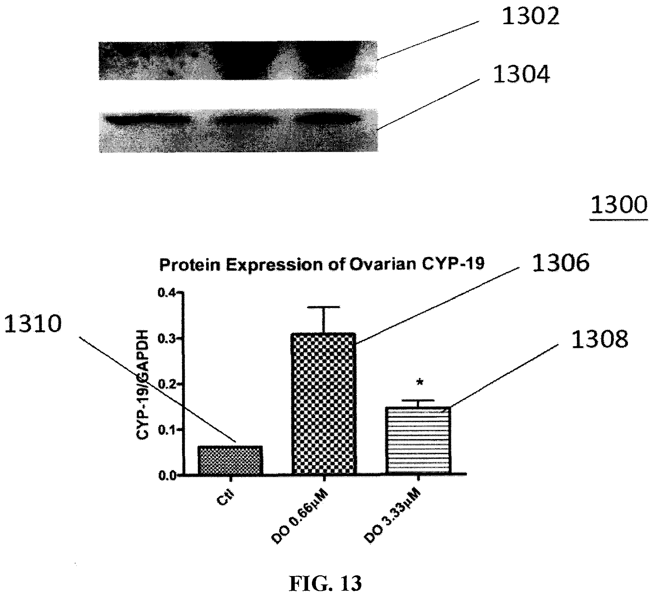

Protein expression of ovarian CYP-19 aromatase (also known as estrogen synthetase) was compared to expression of glyceraldehyde 3-phosphate dehydrogenase (GAPDH) in granulosa cells treated with DO peptide. Protein was extracted from granulosa cells and ovaries of Sprague-Dawley rats using RIPA buffer (Sigma Aldrich) with Complete Protease Inhibitor Cocktail Tablets (Roche Applied Science). 20 µg of denatured proteins per sample were separated on SDS-PAGE and transferred to PVDF membranes. Immunoblotting was performed using specific anti-FSHR (follicle stimulating hormone receptor) and anti-ovarian aromatase antibodies, with anti-GAPDH antibody as an internal standard, followed by incubation with horseradish peroxidase-conjugated secondary antibody. Chemiluminescence detection (GE Bio-health, Princeton, NJ) was accomplished with the Bio-Rad Chemi Doc™ EQ densitometer (Bio-rad) and quantified by Bio-Rad Quantity One software (Bio-Rad laboratories, Hercules, USA). Results are shown in FIG. 13.

Specific induction of the CYP-19 protein compared to GAPDH is apparent from a comparison of the thickness of bands 1302 (CYP-19) and 1304 (GAPDH) obtained from SDS PAGE. The protein expression of ovarian CYP-19 / GAPDH in granulosa cells treated with DO at 0.66µM (1306) and 3.33µM (1308) was 0.309 ± 0.059 and 0.146 ± 0.016 respectively, as shown in graph 1300. There was a significant increase in the protein expression in 3.33µM DO peptide treated group 1308 (*p* = *0.0347,* un-paired *t-test*) compared with control group 1310 (0.062 ± 0.001).

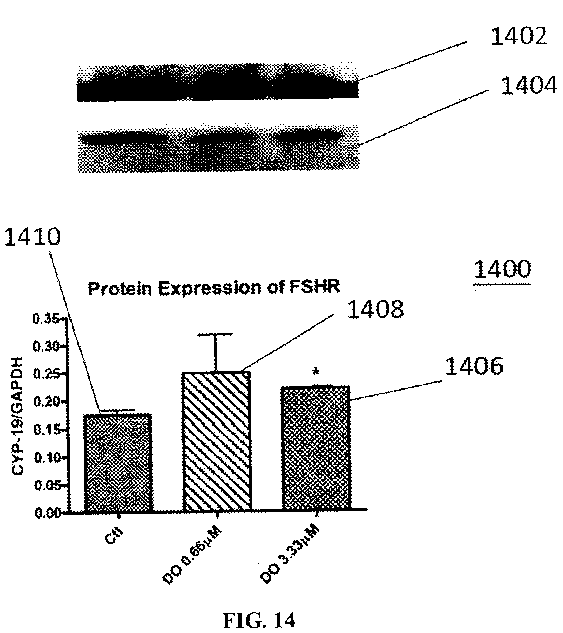

Similarly, expression of follicle-stimulating hormone receptor (FSHR) and GAPDH in granulosa cells treated with DO peptide at 0.66µM and 3.33µM was measured. The results, shown in FIG. 14, indicate that FSHR expression (band 1402) increased compared to GAPDH expression (band 1404). Expression of FSHR/GAPDH in granulosa cells treated with DO protein at 0.66 µM (1408) and 3.33µM (1406) was 0.249 ± 0.069 and 0.220 ± 0.002 respectively. There was a significant increase in the protein expression in the 3.33µM DO peptide treated group 1406 *(p = 0.0391,* un-paired *t-test)* compared with the control group 1410 (0.174 ± 0.009) as shown in Fig. 14.

### RNA extraction and real time PCR

Total RNA was extracted from rat ovaries using a High Pure RNA Isolation Kit (Roche Applied Science) following manufacturer's instruction. cDNA was synthesized from total RNA using a First Strand cDNA Synthesis Kit (Fermentas) following manufacturer's instruction. Real time PCR was performed using a LightCycler 480 Real-Time PCR system (Roche Applied Science).

Primers for ovarian CYP-19 (Forward: 5' GAGAGTTCATGAGAGTCTGGATCA (SEQ ID NO:2), Reverse: 5' GATATAGTTGCTGTGCTTCATCA (SEQ ID NO:3)).

Primers for FSHR (Forward: 5' GAAAGGATCATTTGCTGGATTT (SEQ ID NO:4), Reverse: 5' CTTCCAAGACATCATTCTGAGAGA (SEQ ID NO:5)).

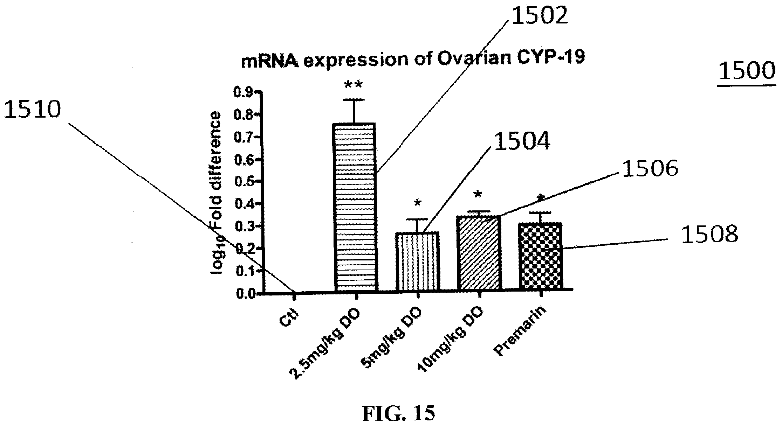

The mRNA expression level of ovarian CYP-19 aromatase in ovaries from Sprague-Dawley rats treated with DO peptide or Premarin was determined. The animals were treated for 6 weeks; DO peptide was administered by intraperitoneal injection, and Premarin was given orally. A control group of animals received phosphate buffered saline (PBS) by intraperitoneal injection. The results are presented in graph 1500 in FIG. 15. Expression of mRNA for ovarian CYP-19 aromatase in rats treated with 2.5 mg/kg (1502), 5 mg/kg (1504), and 10 mg/kg (1506) of DO peptide was 0.751 ± 0.108, 0.257 ± 0.062, and 0.327 ± 0.025, respectively, expressed as log₁₀ fold difference. Expression of mRNA in animals given Premarin (12.4mg/kg daily), 1508, was 0.290 ± 0.051log₁₀ fold difference. There was a significant increase in the mRNA expression level in 1502 (2.5 mg/kg) (p < *0.01*), 1504 (5 mg/kg) (*p* < *0.05*), 1506 (10 mg/kg) (p < *0.05*) DO peptide treated groups and Premarin treated group 1508 (*p* < *0.05*) compared with control group 1510 by one way ANOVA statistical analysis followed by a Dunnett post-test.

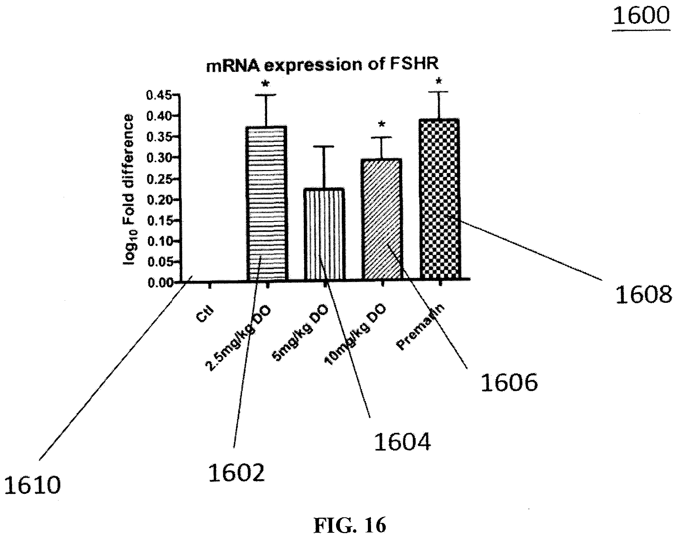

Expression of mRNA for FSHR was measured in Sprague-Dawley rats after 6 weeks of treatment with DO peptide. As shown in graph 1600 of FIG. 16, the mRNA expression level of FSHR in ovaries from Sprague-Dawley rats treated with DO peptide at 2.5 mg/kg (1602), 5 mg/kg (1604), 10 mg/kg (1606) and Premarin at 12.4mg/kg (1608) was measured and expressed as log₁₀ fold difference. The mRNA expression levels were 0.370 ± 0.078 (1602), 0.221 ± 0.101 (1604), 0.288 ± 0.053 (1606) and 0.382 ± 0.068 (1608) respectively. There was a significant increase in the mRNA expression level in groups 1602 (*p* < *0.05*), 1606 (p < *0.05*), and Premarin treated group 1608 (p < *0.05*) compared with control group 1610. Statistical analysis was performed using one-way ANOVA followed by a Dunnett post-test.

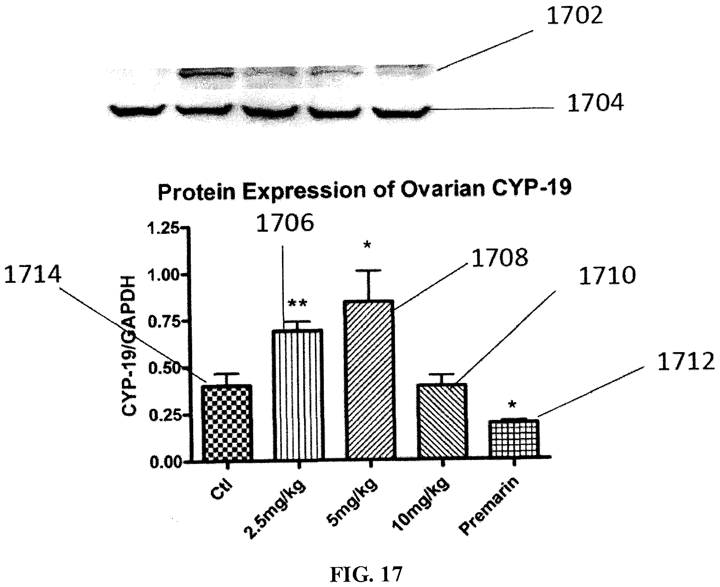

The protein expression level of ovarian CYP-19 aromatase was measured and compared to expression of GAPDH in ovaries from Sprague-Dawley rats treated with DO peptide. DO peptide was administered to female Sprague-Dawley rats at 2.5 mg/kg, 5 mg/kg, 10 mg/kg daily for 6 weeks. A control group of animals received 12.4mg/kg Premarin daily for 6 weeks. A negative control group of animals received PBS injections daily for 6 week. The results are shown in FIG. 17. CYP-19 1702 and GAPDH 1704 expression were analyzed using SDS PAGE. The ratio of CYP-19/GAPDH was determined to be 0.691 ± 0.048 (1706), 0.842 ± 0.167 (1708), 0.391 ± 0.058 (1710) and 0.192 ± 0.015 (1712) for doses 2.5 mg/kg, 5 mg/kg, 10 mg/kg, and Premarin, respectively. There was a significant increase in the protein expression level in 2.5 mg/kg and 5 mg/kg DO treated groups 1706 (p = *0.0051*) and 1708 (*p* = *0.0326*), un-paired *t-test*) compared to control group 1714 (0.398 ± 0.066). There was a significant decrease in Premarin treated group 1712 (*p* = *0.0128,* un-paired *t-test*) compared with control group 1714.

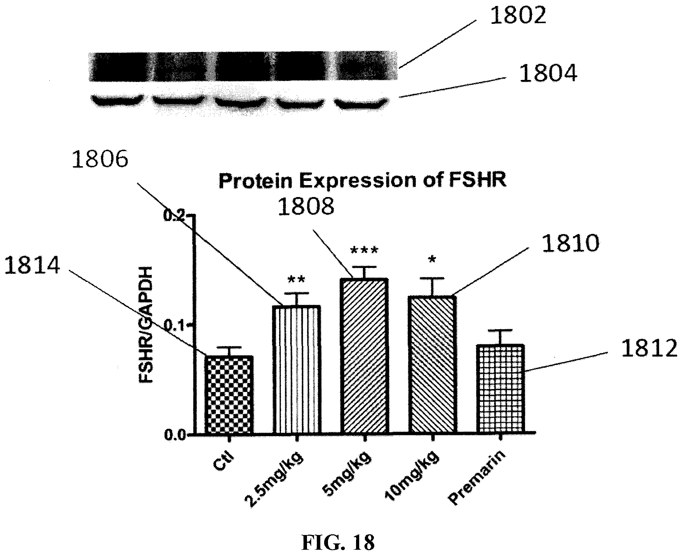

FSHR expression was measured and compared to expression of GAPDH in the ovaries of Sprague-Dawley rats after a 6 week treatment period with DO peptide, Premarin, or PBS. The results are shown in FIG. 18. The protein expression level of FSHR (1802) / GAPDH (1804) in ovaries from rats treated with DO peptide at 2.5 mg/kg (1806), 5 mg/kg (1808), 10 mg/kg (1810) and Premarin (1812) was 0.116 ± 0.012, 0.141 ± 0.012, 0.124 ± 0.017 and 0.079 ± 0.014 respectively. There was significant increase in the protein expression level in 1806 (2.5 mg/kg), 1808 (5 mg/kg) and 1810 (10 mg/kg) DO peptide treated groups (*p* = *0.0099, 0.0004 and 0.0167* respectively, un-paired *t-test*) compared with control group 1814 (0.071 ± 0.009).

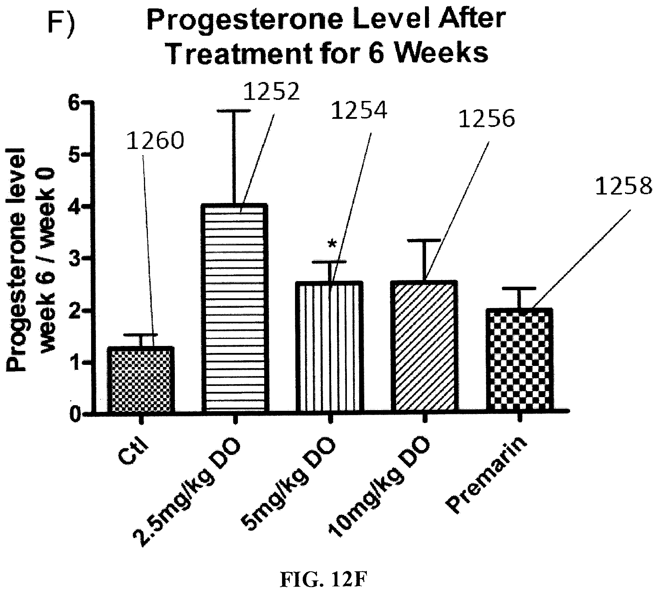

In one embodiment, the DO peptide isolated from Chinese yam tuber (*Dioscorea opposita*) increases the endogenous biosynthesis of estrogen and progesterone by up-regulating synthesis of follicle stimulating hormone receptor (FSHR) (FIGS. 14, 16, 18) and ovarian CYP-19 aromatase (FIG. 13, 15, 17). Both estrogen (FIG. 8, 12) and progesterone levels (FIG. 12) are maintained at premenopausal levels. In addition, progesterone and estrogen synthesis, mediated by FSHR and CYP-19 aromatase, are regulated by the feedback mechanism of the hypothalamus-pituitary axis. In one embodiment menopausal syndrome is relieved by slowing the decline of serum estrogen and progesterone levels (Fig. 12).

### MTT Assay

The MTT Cell Proliferation Assay is a quantitative, convenient method for evaluating a cell population's response to external factors. Results may show an increase in cell growth, no effect, or a decrease in growth due to necrosis or apoptosis.

BT-483 and OVCA-429 cancer cells were serum starved for 24 hours prior to drug treatment. The DO peptide was then added in complete growth medium at the concentration of 1 nM, 10 nM and 100 nM for 48 hours, followed by incubation with MTT solution for 3 hours. Formazan crystals were dissolved by DMSO. Absorbance at O.D. 540 nm was measured with a microplate reader (Model 680, Bio-Rad). Percentage proliferation relative to the control was calculated.

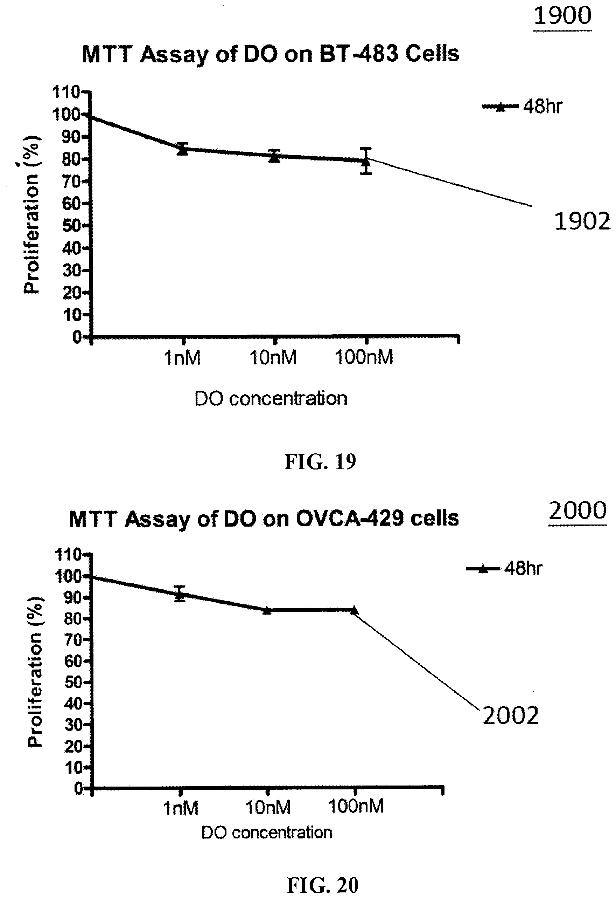

In one embodiment, DO peptide inhibited the proliferation of both BT-483 breast cancer cells (FIG. 19) and OV-429 cells (FIG. 20) in a dose dependent fashion. As indicated by point 1902 in graph 1900, incubation of BT-483 cells with DO peptide at a concentration of 100 nM for 48 hours decreased the proliferation of BT-483 breast cancer cells to 78.65 ± 5.502% of control value. As shown in graph 2000, incubation of OV-429 ovarian cancer cells decreased cellular proliferation to 83.81 ± 1.899% (2002).

### Effect of DO Peptide on Body Weight

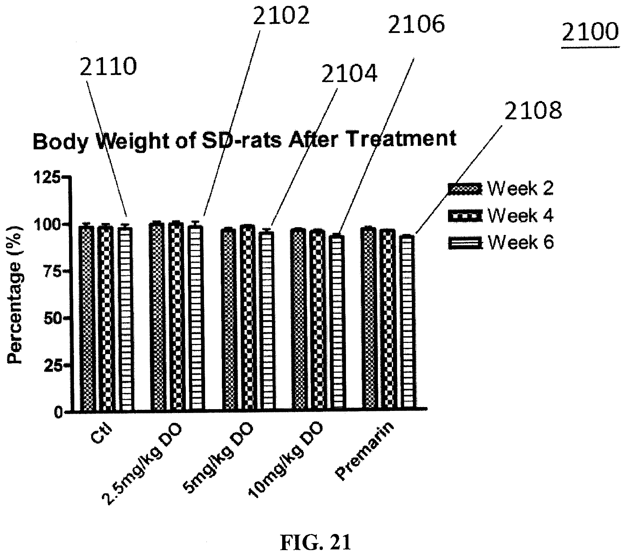

The body weights of female Sprague-Dawley rats given either DO peptide or Premarin were determined after 6 weeks of treatment. DO peptide was administered at doses of 2.5 mg/kg, 5 mg/kg, 10 mg/kg, and Premarin at a dose of 12.54 mg/kg. Results are shown in graph 2100, FIG. 21. Body weights after 2, 4, and 6 weeks of treatment were compared with the pre-treatment value. The final body weights after 6 weeks of treatment were found to be 95.27 ± 3.76% (2102), 90.78 ± 4.15%, (2104), 92.17 ± 1.46% (2106) for DO peptide doses of 2.5 mg/kg, 5 mg/kg, 10 mg/kg, respectively, and 91.72 ± 0.98% (2108) for Premarin. There was no significant difference with the control group (2110) (97.31 ± 2.32) and any of the treated groups.

### Measurement of bone status: Bone mineral density and micro-architecture

### Sample Preparation

Lumbar vertebrae including soft tissue were harvested from Sprague-Dawley rats after 6-weeks of treatment with either DO peptide or Premarin. The samples were harvested, wrapped in normal saline gauze (0.9%), and stored at -80 degree Celsius. The specimens were thawed at room temperature prior to evaluation of bone status by In Viva MicroCT 40 computed tomography system (Scanco Medical, Basserdorf, Switzerland).

### Image Acquisition

The lumbar vertebrae wrapped with saline gauze were placed in a sample holder and its long axis aligned with the axis of rotation of the X-ray gantry. A scout view was obtained and was used to identify the 2nd lumbar vertebra (L2) and to determine the scanning location. A set of 100 contiguous axial slices at an isotropic resolution of 21 µm was prescribed at the L2 mid-vertebral body. Exposure parameters were set at X-ray tube peak voltage of 70 kVp and tube current of 114 µA with integration time of 300 ms. Calibration of X-ray attenuation to bone mineral density (mg/cc of hydroxyapatite (HA)) with the microCT scanner was carried out weekly using a standardized phantom.

### Image processing and analysis

A Gaussian filter with a support =2 and sigma =1.2 was used to reduce the image noises. Precise contouring was performed in each of the slices by drawing the region of interest manually a few voxels away from the endocortical boundary. This was then followed by segmentation using a global threshold of 250 for all the study groups in order to separate the mineralized tissues from non-mineralized tissues.

Volume rendering of these segmented slices was performed to provide a three-dimensional (3D) image. The apparent trabecular bone mineral density and its bone micro-architecture were automatically evaluated using the built-in program of the microCT with the model independent direct 3D morphometry. The bone micro-architecture including apparent trabecular bone mineral density, bone volume fraction, trabecular number, trabecular thickness, trabecular separation and structural model index were reported.

Table 3 lists the bone micro-architecture parameters used to evaluate bone mineralization and provide a measure of anti-osteoporotic activity.

**Table 3**

| Abbreviation | Variable | Description | Standard unit |
|---|---|---|---|
| tBMD | Apparent trabecular bone mineral density | Bone mineral density of the total bone volume including the bone marrow | mg/cc HA |
| BV/TV | Bone volume fraction | Ratio of the segmented bone volume to the total volume of the region of interest | % |
| Tb.N | Trabecular number | Measure of the average number of trabecular per unit length | 1/mm |
| Tb.Th | Trabecular thickness | Mean thickness of trabeculae, assessed using direct 3D methods | mm |
| Tb.Sp | Trabecular separation | Mean distance between trabeculae, assessed using direct 3D methods | mm |
| SMI | Structure model index | An indicator of the structure of trabeculae; SMI will be 0 for parallel plates and 3 for cylindrical rods | |

The anti-osteoporotic activity of DO peptide was evaluated in female Sprague-Dawley rats by comparing the bone calcification effects of DO peptide to the hormone replacement drug, Premarin and Raloxifene. Raloxifene is a drug that prevents and treats osteoporosis by mimicking the effects of estrogen to increase the density (thickness) of bone. Premarin, and Raloxifene were used as positive controls.

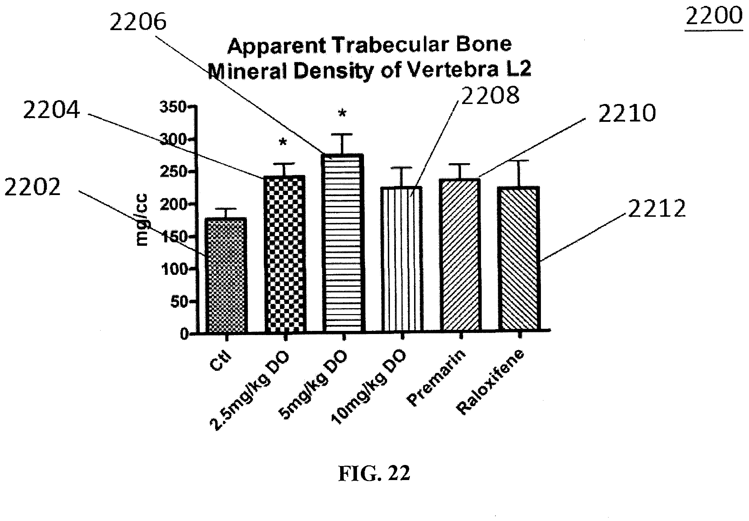

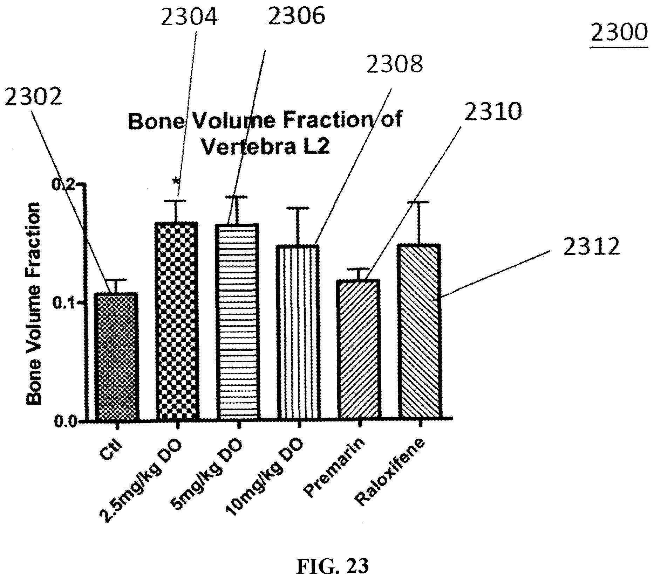

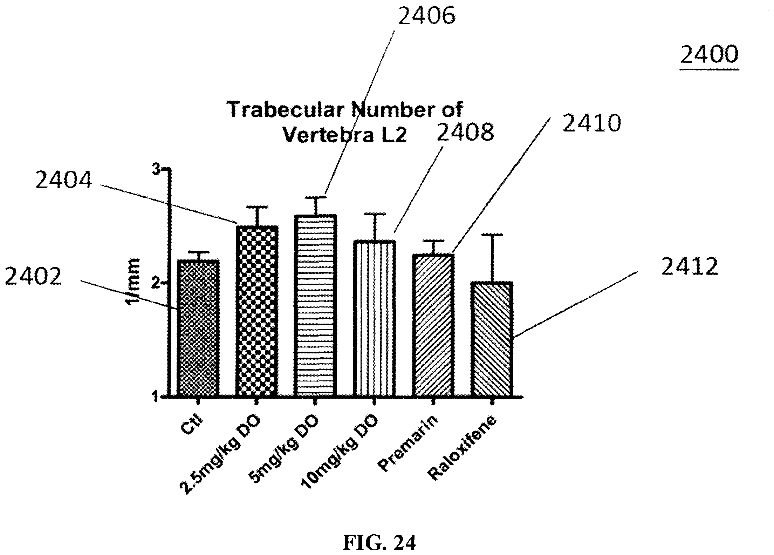

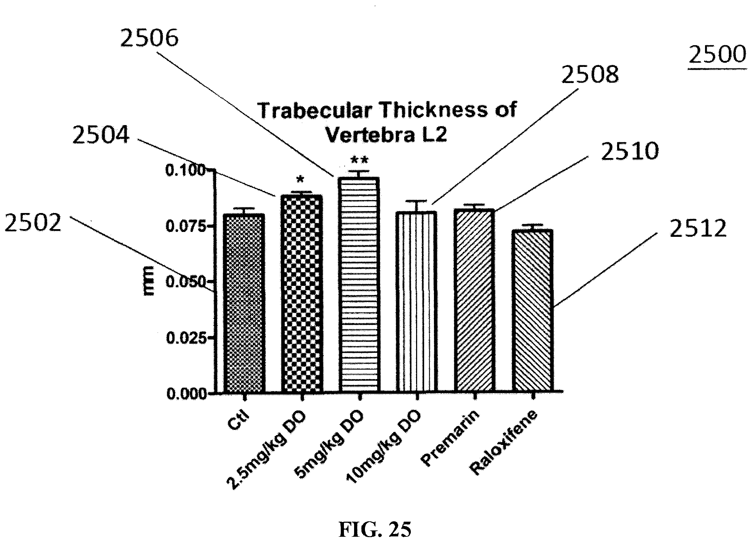

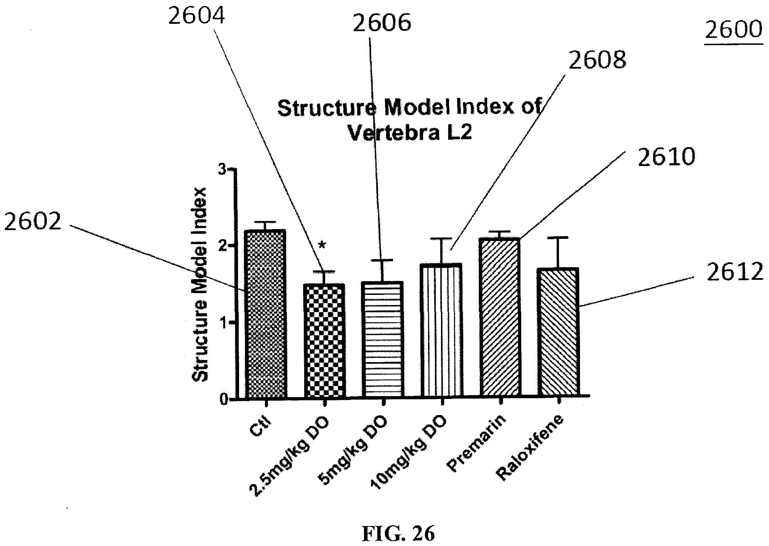

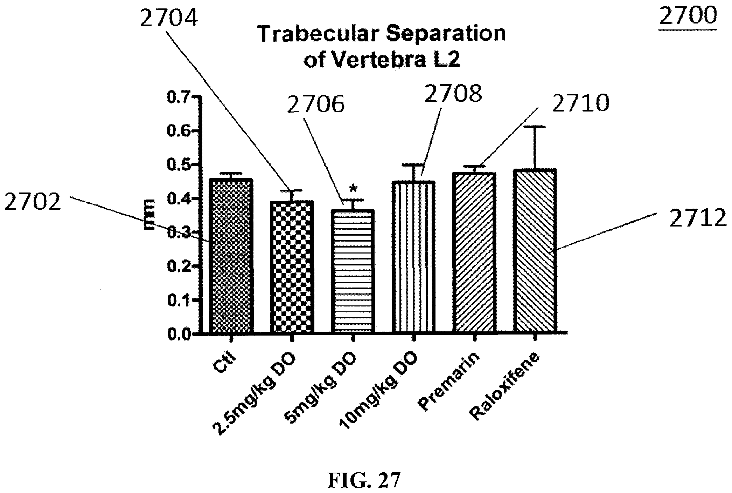

Groups of 3-6 animals per group were dosed daily with 2.5 mg/kg 5 mg/kg, 10 mg/kg of DO peptide, 12.4 mg/kg Premarin, and 25.56 mg/kg Raloxifene. A negative control group received daily doses of PBS. After 6 weeks of dosing, the L2 vertebra of the animals was harvested and examined for apparent trabecular bone mineral density (FIG. 22), bone volume fraction (FIG. 23), trabecular number (FIG. 24), trabecular thickness (FIG. 25), structure model index (FIG. 26), and trabecular separation (FIG. 27).

The mean apparent trabecular bone mineral density (tBMD) of vertebra L2 in the groups treated with DO peptide are shown in graph 2200 (FIG. 22). Animals given 2.5 mg/kg, 5 mg/kg, and 10 mg/kg DO peptide had tBMD measurements of 240.2 ± 21.03 mg/cc HA (2204), 272.5 ± 32.2 mg/cc HA (2206), and 222.1 ± 30.24 mg/cc HA (2208) respectively. Premarin treated animals had tBMD measurement of 233.6 ± 24.23 mg/cc HA (2210) and Raloxifene treated animals had a tBMD measurement of 219.4 ± 42.36 mg/cc HA (2212). There was a significant increase in tBMD in 2.5 mg/kg (2204) and 5 mg/kg (2206) DO treated groups (*p* = *0.0351* and *0.0229* respectively, un-paired *t-test*) compared to negative control group (2202) (176.3 ± 15.69 mg/cc HA).

Bone volume measurements are presented in graph 2300 (FIG. 23). The percentage bone volume fraction (BV/TV) of vertebra L2 were 16.57 ± 1.939%, 16.43 ± 2.378%, 14.59 ± 3.228%, for animals given DO peptide at doses of 2.5 mg/kg (2304), 5 mg/kg (2306), 10 mg/kg (2308), respectively. Premarin treated group 2310 had a bone volume fraction of 11.61 ± 0.996%, and Raloxifene treated group 2312 was 146.1 ± 3.61%. There was a significant increase in bone volume fraction in 2.5 mg/kg DO treated group (*p* = *0.0275,* un-paired *t-test*) compared to control group 2302 (10.73 ± 1.167%).

Trabecular bone numbers (Tb.N) of vertebra L2 are presented in graph 2400 (FIG. 24). The trabecular bone numbers were 2.491 ± 0.178 mm⁻¹, 2.59 ± 0.164 mm⁻¹, 2.365 ± 0.243 mm⁻¹, for animals given DO peptide at doses of 2.5 mg/kg (2404), 5 mg/kg (2406), 10 mg/kg (2408), respectively. Premarin treated group 2410 had a trabecular bone number of, 2.247 ± 0.124 mm⁻¹, and Raloxifene treated group 2412 was 2 ± 0.425 mm⁻¹. There was an average increase of 13.59%, 18.10% and 7.84% in Tb.N the groups 2404, 2406, and 2408 treated daily with DO peptide at 2.5 mg/kg (2404) 5 mg/kg (2406) and 10 mg/kg (2408) respectively compared to control group 2402 (2.193 ± 0.082 mm⁻¹), which is not statistically different from that of Premarin treated group 2410.

Trabecular thickness (Tb.Th) measurements of vertebra L2 are presented in graph 2500, shown in FIG. 25. The Tb.Th measurements were 0.088 ± 0.002 mm (2504), 0.096 ± 0.003 mm (2506), and 0.0801 ± 0.005 mm (2508) for animals given DO peptide at doses of 2.5 mg/kg (2504), 5 mg/kg (2506), 10 mg/kg (2508), respectively. The Premarin treated control group had a Tb.Th measurement 2510 of 0.081 ± 0.003 mm, and the Raloxifene treated group had Tb.Th measurement 2512 of 0.0719 ± 0.003 mm. There was a significant increase in Tb.Th 2104 and 2506 in the 2.5 mg/kg and 5 mg/kg DO treated groups (*p* = *0.0419* and *0.0052* respectively, un-paired *t-test*) compared to control group 2502 (0.080 ± 0.003 mm).

The structure model indices (SMI) of the L2 vertebrae are presented in graph 2600, shown in FIG. 26. The SMI values of vertebra L2 were 1.48 ± 0.177 (2604), 1.501 ± 0.281 (2606), and 1.715 ± 0.352 (2608) for animals given DO peptide at doses of 2.5 mg/kg (2604), 5 mg/kg (2606), 10 mg/kg (2608), respectively. The Premarin treated control group had SMI value (2610) of 2.047 ± 0.101, and Raloxifene treated control group 2612 had an SMI value of 1.653 ± 0.402. There was a significant decrease in SMI value 2604 of the 2.5 mg/kg DO treated group (*p* = *0.0115,* un-paired *t-test*) compared to control group SMI value 2602 of 2.186 ± 0.121.

The trabecular separations (Tb.Sp) of the L2 vertebrae are presented in graph 2700, shown in FIG. 27. The Tb.Sp values were 0.388 ± 0.034 mm (2704), 0.360 ± 0.033 mm (2706), and 0.445 ± 0.051 mm (2708) for animals given DO peptide at doses of 2.5 mg/kg, 5 mg/kg, 10 mg/kg, respectively. The Premarin treated control group had Tb.Sp value 2710 of 0.471 ± 0.021 mm; the Raloxifene treated group had Tb.Sp value 2712 of 0.480 ± 0.127 mm. There was an significant decrease in Tb.Sp value 2706 in the 5 mg/kg DO treated group (*p* = *0.0358,* un-paired *t-test*) compared to the negative control group Tb.Sp value 2702 of 0.454 ± 0.020 mm.

High resolution microCT reveal a general increase in bone mineral density (Fig 22), bone volume fraction (Fig 23), trabecular number (Fig 24) and trabecular thickness (Fig 25), and a concomitant decrease in both structure model index (Fig 26) and trabecular separation (Fig 27). These density and bone micro-architecture changes favor an enhancement of bone strength. The decrease in structure model index indicates an increase in the ratio of plate-shaped to rod-shaped trabeculae. The latter trabecular structure is dominant in osteoporosis. The trabeculae shape changes show that the bone may become less porous as reflected in the parallel decrease in trabecular separation. In one embodiment, administration of DO peptide exerts an anti-osteoporotic effect on the trabecular bone in this old-age rat model.

### Measurement of Brain Derived Neurotrophic Factor (BDNF) translational level in hippocampus and Prefrontal cortex

The translational level of BDNF in rats' hippocampus and prefrontal cortex was measured by Brain Derived Neurotrophic Factor (BDNF) Sandwich ELISA Kit (Cat. No. CYT306; Millipore, Inc., Billerica, MA, USA), following the manufacturer's instruction. The translational levels of BDNF were normalized by the total protein concentration of the individual samples.

Groups of 3-6 animals per group were dosed daily with 2.5 mg/kg 5 mg/kg, 10 mg/kg of DO peptide, or 12.4 mg/kg Premarin. A negative control group received daily doses of PBS. After 6 weeks of dosing, BDNF protein levels (normalized to total protein content) were determined in the hippocampus, and prefrontal cortex.

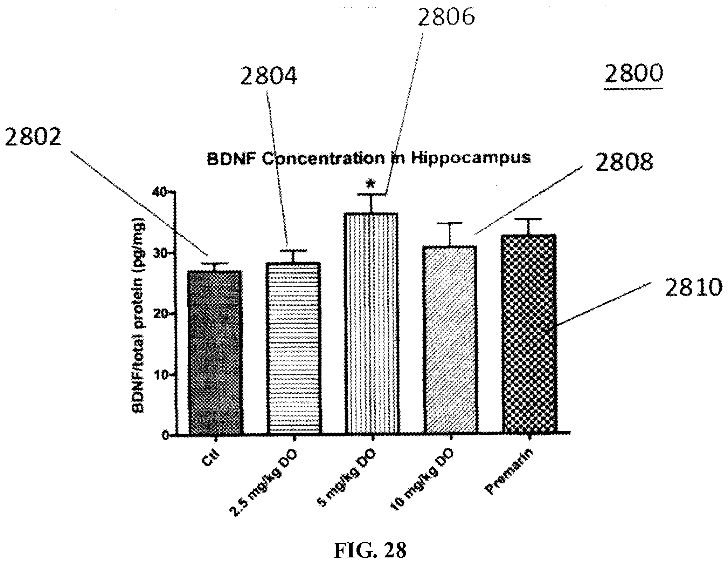

The BDNF levels in the hippocampus, presented in graph 2800 (FIG. 28), were 28.10 ± 2.117 pg/mg, 36.17 ± 3.177 pg/mg, 30.70 ± 3.878 pg/mg for animals given DO peptide at doses of 2.5 mg/kg (2804), 5 mg/kg (2806), 10 mg/kg (2808), respectively. The Premarin treated control group 2810 had BDNF protein concentration 2810 32.46 ± 2.749 pg/mg in the hippocampus. There was a significant increase in the BDNF protein level in group 2806 that had received 5 mg/kg DO peptide (*p* = *0.024,* un-paired *t-test*) compared with the control group 2802 (26.87 ± 1.392 pg/mg).

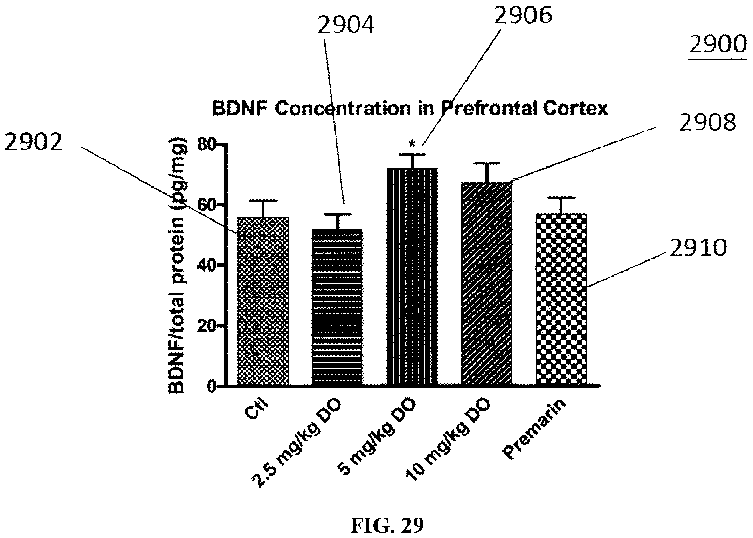

The BDNF levels in the prefrontal cortex, presented in graph 2900 (FIG. 29) were 51.87 ± 4.942 pg/mg, 71.82 ± 4.682 pg/mg, 67.22 ± 6.331 pg/mg for animals given DO peptide at doses of 2.5 mg/kg (2904), 5 mg/kg (2906), and 10 mg/kg (2908), respectively. The Premarin treated control group had BDNF protein concentration 56.84 ± 5.478 pg/mg (2910) in the prefrontal cortex. There was significant increase in the BDNF protein level 5 mg/kg DO treated group 2906 (*p* = *0.047,* un-paired *t-test*) compared with control group 2902 (55.77 ± 5.580).

TrkB is the high affinity catalytic receptor for several neurotrophins, which are small protein growth factors that induce the survival and differentiation of distinct cell populations. The neurotrophins that activate TrkB include BDNF. TrkB mediates the multiple effects BDNF and other neutrophins including neuronal differentiation and survival within the brain.

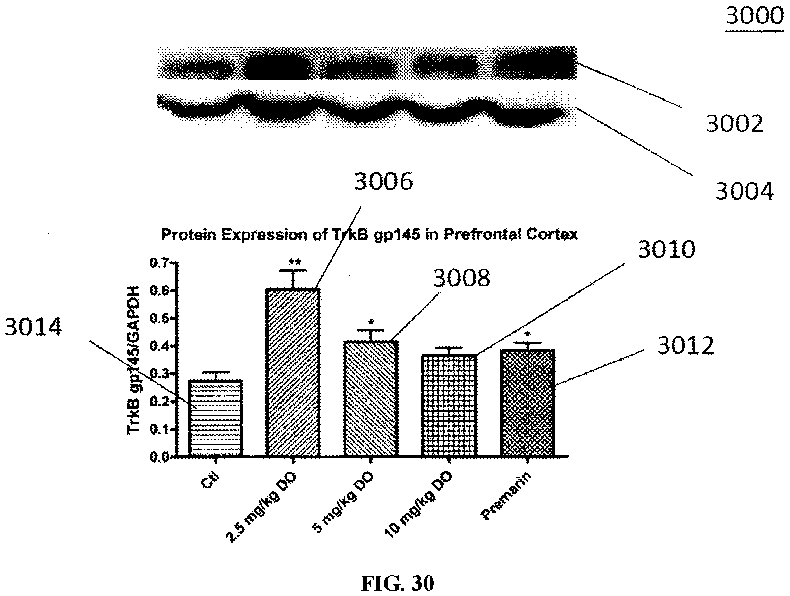

Specific induction of TrkB gp145 receptor protein compared to GAPDH 3004 is apparent from a comparison of the thickness of bands 3002 (TrkB gp145) and 3004 (GAPDH) obtained from SDS PAGE, shown in FIG. 30. The protein levels of TrkB gp145 receptor/GAPDH in the prefrontal cortex, presented in graph 3000, FIG. 30 were 0.6038 ± 0.06977, 0.4150 ± 0.04169, 0.3638 ± 0.02919 for animals given DO peptide at doses of 2.5 mg/kg (3006), 5 mg/kg (3008), and 10 mg/kg (3010), respectively. The Premarin treated control group had TrkB gp145 receptor/GAPDH ratio of 0.3805 ± 0.03002 (3012) in the prefrontal cortex. There was a significant increase in the protein expression level in 3006 (2.5 mg/kg), 3008, 5 mg/kg and Premarin 3012 treated groups (*p* = *0.0016, 0.0239 and 0.0377* respectively, un-paired *t-test*) compared with control group 3014 (0.2723 ± 0.03377).

In one embodiment, DO peptide exerts beneficial effects on cognitive functions, as revealed by the elevated BDNF translational level in the DO-treated rats (Fig 28). BDNF is involved in synaptic plasticity and both maintenance and survival of neurons in the hippocampus and cortex, which is associated with cognitive functions. While BDNF expression decreases in deprivation of estrogen, estrogen replacement in rodents can reverse the change. The elevated protein expression level of BDNF and TrkB receptor in the prefrontal cortex of DO peptide treated rats (FIGs. 29, 30) indicates that the DO peptide has a stimulatory effect in prefrontal BDNF signaling and a potential beneficial role in enhancing cognitive functions during aging.

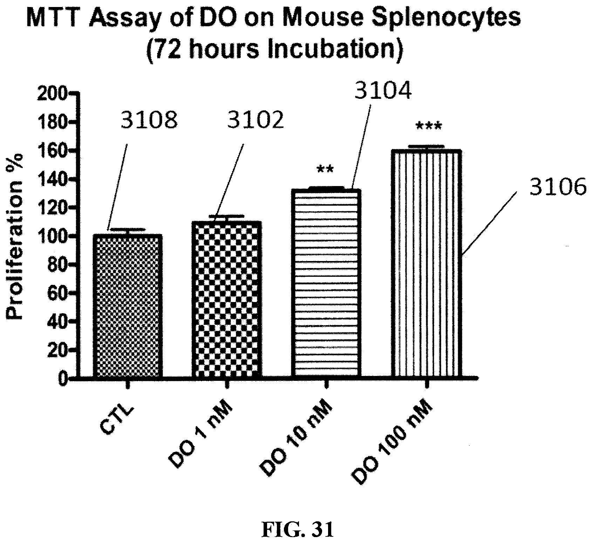

In one embodiment, the mitogenic activity of DO was assayed using mouse splenocytes. Splenocytes were isolated from BALB/c mice. The isolated splenocytes were diluted with RPMI 1640 with 15% fetal bovine serum and 1% penicillin-streptomycin and seeded in 96-well microplates at a density of 5 × 10⁵ cells / 100 µl /well. Cells were cultured at 37°C in a humidified atmosphere with 5% carbon dioxide for 24 hours. DO peptide was then added at 1 nM (3102), 10 nM (3104) and 100 nM (3106) concentrations, as shown in FIG.31. Cells without DO treatment served as control 3108. The cells after adding drug were incubated for further 72 hours, followed by incubation with MTT solution for 3 hours. Formazan crystals were dissolved by DMSO. Absorbance at O.D. 540 nm was measured with a microplate reader (Model 680, Bio-Rad). Percentage proliferation relative to the control was calculated.

As shown in the FIG. 31, DO peptide displayed a mitogenic effect, as reflected by the proliferation of mouse splenocytes, in a dose dependent manner. There were a significant increase in proliferation percentage in mouse splenocytes treated with 10 nM (3104) and 100 nM (3106) DO peptide, where the mean proliferation percentage relative to control 3108 were 131.6 ± 2.098% (*p*=*0.0026,* unpaired t-test) and 158.8 ± 3.610% (*p*<*0.0001,* unpaired t-test) (mean ± SEM) respectively.

Menopause and general ageing are accompanied by the decline in immune functions. Changes in the immune system during aging including immune tolerance, increase of autoantibody reactions, decline in function of natural killer cells, B lymphocytes and T lymphocytes have been observed. Reduced estrogen levels after menopause correlate with increased levels of pro-inflammatory serum markers, decreased in CD4 T and B lymphocytes levels. In one embodiment, administration of DO peptide improves immune function in aged individuals through a mitogenic effect on splenocytes.

### Purification of the DO Protein Using Antibody - Affinity Column Purification Method

Basic antigen affinity-purified polyclonal antibody (rabbit) was raised with purified DO protein as the antigen (GenScript). The anti-DO antibody was covalently attached to a column with the use of AminoLink® Plus Immobilization kit (Thermo Scientific) to produce an affinity column that purifies the DO protein by the affinity to the antibody.

*Dioscorea opposita* was peeled and homogenized in an aqueous extraction buffer (5% acetic acid + 0.1% β-mercaptoethanol) in a ratio of 1:2 (w/v) for 3 hours at 4°C. The homogenate was subjected to centrifugation at 17,700g for 30 min at 4°C. The supernatant was collected and ammonium sulfate was added to 80% of saturation. The mixture was stirred at 4°C overnight and subjected to centrifugation at 17,700g for 1 hr at 4°C. The precipitate (protein extract) was retained and resuspended in purified water. The protein extract mixture was dialyzed against doubly distilled H₂O overnight and then subjected to ultra-centrifugation at 40,000g for 2 hr at 4°C. The supernatant was collected and subjected to the affinity column purification according to the manufacturer instructions of AminoLink® Plus Immobilization kit.

Estrogen concentration in cell culture medium of DO-treated primary cell culture of rat ovarian granulosa cells was measured. Briefly,_21 to 23 day-old female Sprague-Dawley (SD)-rats were primed with 15 IU PMSG (Sigma) for 48 hours to stimulate follicular development. The rats were then sacrificed and ovaries were dissected. The ovarian follicles were punctured with a 25-gauge needle. The isolated granulosa cells were cultured at 37°C in an atmosphere of 5% CO₂ in culture medium consisting of serum free DMEM/F12 1:1 medium containing 1% penicillin-streptomycin, 0.1% BSA and insulin (1 µg/ml) for 24 hour.

The DO protein was isolated from various *Dioscorea* species isolated using AminoLink® Plus Column linked to anti-DO antibody was then added to the granulosa cells for 24-hour treatment. The cell culture medium was collected for measurement of estradiol concentration and the cells were harvest for protein extraction. Protein concentration from cellular lysate was determined with Bradford assay.

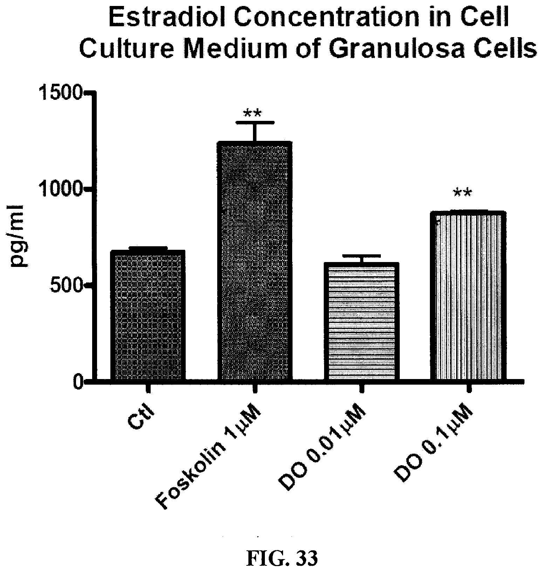

**Fig. 33** shows estradiol concentration in cell culture medium of granulosa cells. The estradiol concentration of granulosa cell medium is expressed as mean pg estradiol / ml ± SEM (n=3). The estradiol levels in control (Ctl) group, treatment with forskolin 1µM, DO 0.01µM and DO 0.1µM are respectively 671.0 ± 23.70, 1236 ± 108.0, 872.9 ± 10.33 and 609.2 ± 44.82 pg/ml. There is significant difference in forskolin 1µM (*p*<*0.01*) and DO 0.1µM (*p*<*0.01*) group compared with control group in unpaired *t-test.*

The DO protein stimulates expression of ovarian aromatase and elevates serum estradiol level. This Example investigates the safety and efficacy of the DO protein for relieving menopausal symptoms. The activation of aromatase in extragonadal tissues such as mammary gland may lead to pathological consequences. Also, hyperactivation of aromatase in breast tissue may promote the progression of breast cancer, leading to enhanced estrogen production for growth of breast tumor.

To evaluate the effect of DO protein on stimulating aromatase expression in extragonadal tissue, protein expression of aromatase in mammary gland was determined by Western blotting analysis. Protein expression of CYP-19 aromatase in mammary glands was compared to expression of glyceraldehyde 3-phosphate dehydrogenase treated with the DO peptide.

Briefly, proteins were extracted from mammary glands of Sprague-Dawley rats using RIPA buffer (Sigma Aldrich) with Complete Protease Inhibitor Cocktail Tablets (Roche Applied Science). 20 µg of denatured proteins per sample were separated on SDS-PAGE and transferred to PVDF membranes. Immunoblotting was performed using specific anti-aromatase antibodies, with anti-GAPDH antibody as an internal standard, followed by incubation with horseradish peroxidase-conjugated secondary antibody. Chemiluminescence detection (GE Bio-health, Princeton, NJ) was accomplished with the Bio-Rad Chemi Doc™ EQ densitometer (Bio-rad) and quantified by Bio-Rad Quantity One software (Bio-Rad laboratories, Hercules, USA).

Expression of protein for aromatase in mammary glands was measured in Sprague-Dawley rats after 6 weeks of treatment with DO peptide. The protein expression level of aromatase in mammary glands from Sprague-Dawley rats from control group or treated with DO peptide at 2.5 mg/kg, 5 mg/kg, 10 mg/kg and Premarin at 12.4 mg/kg was measured and expressed mean relative intensity ± standard deviation.

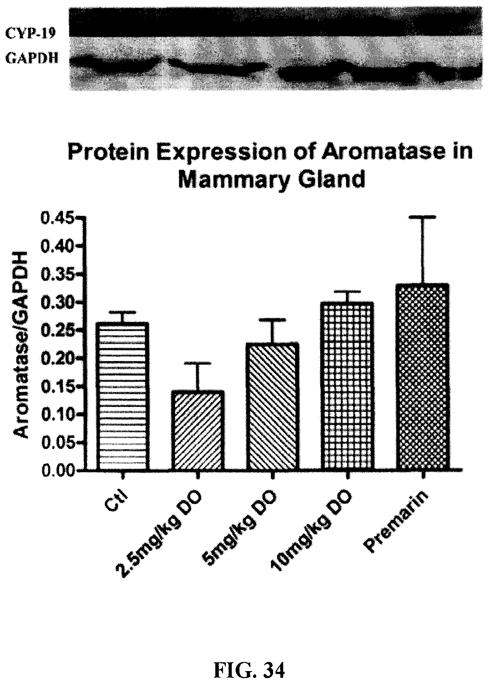

**Fig. 34** shows that the protein expression levels of aromatase were 0.2614 ± 0.04082 (control), 0.1390 ± 0.08948 (2.5 mg/kg DO-treated), 0.2243 ± 0.08658 (5 mg/kg DO-treated), 0.2963 ± 0.04294 (10 mg/kg DO-treated), 0.3285 ± 0.2428 (premarin-treated), respectively. No statistical differences were detected in statistical analysis using one-way ANOVA.

The results show that the DO protein stimulates aromatase in ovaries of Sprague-Dawley rats, but not in mammary glands, indicating that DO has tissue-specific aromatase modulating properties. This shows that the DO protein is less likely to increase the risk of breast cancers due to extragonadal stimulation of aromatase in breast tissues.

### Antibody

In one embodiment, the present invention provides an antibody that binds specifically to a polypeptide of the present invention. In one embodiment, the antibody binds specifically to polypeptide obtainable from *Dioscorea* sp., wherein the polypeptide has an apparent molecular weight of about 32.5 kDA by chromatography, wherein the first twenty-one consecutive amino acids at the N-terminal of the polypeptide consists of SEQ ID NO:1 and the polypeptide increases the level of estrogen *in vitro* or *in vivo.*

An antibody that is contemplated for use in the present invention can be in any of a variety of forms, including a whole immunoglobulin, an antibody fragment such as Fv, Fab, and similar fragments, as well as a single chain antibody that includes the variable domain complementarity determining regions (CDR), and similar forms, all of which fall under the broad term "antibody," as used herein.

The term "antibody fragment" refers to a portion of a full-length antibody, generally the antigen binding or variable region. Examples of antibody fragments include Fab, Fab', F(ab')₂ and Fv fragments. Papain digestion of antibodies produces two identical antigen binding fragments, called the Fab fragment, each with a single antigen binding site, and a residual "Fc" fragment, so-called for its ability to crystallize readily. Pepsin treatment of an antibody yields an F(ab')₂ fragment that has two antigen binding fragments, which are capable of cross-linking antigen, and a residual other fragment (which is termed pFc'). Additional fragments can include diabodies, linear antibodies, single-chain antibody molecules, and multispecific antibodies formed from antibody fragments. As used herein, "antigen binding fragment" with respect to antibodies, refers to, for example, Fv, F(ab) and F(ab')₂ fragments. Of particular importance for binding are the first 110 to 130 amino acids at the N-terminus of the amino acid sequences exemplified herein. Thus, high identity in the N-terminus 110, 115, 120, 125, or 130 amino acids constituting the variable region is preferred. Variant sequences preferably have more than 75%, 90%, or even 95% identity in this region.

The subject invention further comprises fusion constructs wherein the antibody, or fragment thereof, may be fused to one or more additional entities. The additional entity(ies) may be for example linkers, toxins, carriers, solid supports, and/or detectable molecules. In this context the binding portion may consist or consist essentially of the antibody.

"Specific binding" or "specificity" refers to the ability of an antibody or other agent to detectably bind an epitope presented on an antigen, while having relatively little detectable reactivity with other proteins or structures. Specificity can be relatively determined by binding or competitive binding assays, using, *e.g.,* Biacore instruments. Specificity can be exhibited by, *e.g.,* an about 10:1, about 20:1, about 50:1, about 100:1, 10.000:1 or greater ratio of affinity/avidity in binding to the specific antigen versus nonspecific binding to other irrelevant molecules.

"Selectivity" refers to the preferential binding of a protein to a particular region, target, or peptide as opposed to one or more other biological molecules, structures, cells, tissues, etc. For example, selectivity can be determined by competitive ELISA or Biacore assays. The difference in affinity/avidity that marks selectivity can be any detectable preference (*e.g.,* a ratio of more than 1:1.1, or more than about 1:5, if detectable.

If desired, the antibodies produced by the B cells can be modified in any suitable process. For example, the binding affinity of the antibodies can be increased via various methods known in the art. For example, binding characteristics can be improved by direct mutation, methods of affinity maturation, phage display, or chain shuffling within the nucleic acids encoding the antibody molecules. For example, individual residues or combinations of residues can be randomized so that in a population of otherwise identical antigen binding sites, all twenty amino acids are found at particular positions. Binding characteristics can also be improved by methods of affinity maturation. (See, e.g., Yang et al. (1995) J. Mol. Bio. 254, 392-403; Hawkins et al. (1992) J. Mol. Bio. 226, 889-896; or Low et al. (1996) J. Mol. Bio. 250, 359-368 (each of which is hereby incorporated by reference in its entirety, particularly with respect to methods of increasing the binding affinity of antibodies)). Methods known in the art include for example, Marks et al. Bio/Technology, 10:779-783 (1992) describes affinity maturation by VH and VL domain shuffling; random mutagenesis of CDR and/or framework residues is described by Barbas et al. Proc. Natl. Acad. Sci., USA 91:3809-3813 (1994); Schier et al. Gene, 169:147-155 (1995); Yelton et al. J. Immunol., 155:1994-2004 (1995); Jackson et al., J. Immunol., 154(7):3310-3319 (1995); and Hawkins et al, J. Mol. Biol., 226:889-896 (1992).

Strategies for antibody optimization are sometimes carried out using random mutagenesis. In these cases positions are chosen randomly, or amino acid changes are made using simplistic rules. For example all residues may be mutated to alanine, referred to as alanine scanning. WO 9523813 (which is hereby incorporated by reference in its entirety) teaches in vitro methods of increasing antibody affinities utilizing alanine scanning mutagenesis. Alanine scanning mutagenesis can also be used, for example, to map the antigen binding residues of an antibody (Kelley et al., 1993, Biochemistry 32:6828-6835; Vajdos et al., 2002, J. Mol. Biol. 320:415-428). Sequence-based methods of affinity maturation (see, U.S. Pat. Application No. 2003/022240 A1 and U.S. Pat. No. 2002/177170 A1, both hereby incorporated by reference in their entireties) may also be used to increase the binding affinities of antibodies.

Antibodies within the scope of the invention can be of any isotype, including IgG, IgA, IgE, IgD, and IgM. IgG isotype antibodies can be further subdivided into IgG1, IgG2, IgG3, and IgG4 subtypes. IgA antibodies can be further subdivided into IgA1 and IgA2 subtypes.

### Modification of Amino Acid Sequences

If desired, the subject protein can be modified by any suitable process. For example, individual residues or combinations of residues can be randomized so that in a population of otherwise identical antigen binding sites, all twenty amino acids are found at particular positions. Binding characteristics can also be improved by methods of affinity maturation. Strategies for protein optimization are sometimes carried out using random mutagenesis. In these cases positions are chosen randomly, or amino acid changes are made using simplistic rules. For example all residues may be mutated to alanine, referred to as alanine scanning. In addition, substitution of amino acids other than those specifically exemplified or naturally present in a protein of the invention are also within the scope of the present invention. For example, non-natural amino acids can be substituted for the amino acids of the protein, so long as the protein having the substituted amino acids retains substantially the same functional activity as the protein in which amino acids have not been substituted.

Examples of non-natural amino acids include, but are not limited to, ornithine, citrulline, hydroxyproline, homoserine, phenylglycine, taurine, iodotyrosine, 2,4-diaminobutyric acid, α-amino isobutyric acid, 4-aminobutyric acid, 2-amino butyric acid, γ-amino butyric acid, ε-amino hexanoic acid, 6-amino hexanoic acid, 2-amino isobutyric acid, 3-amino propionic acid, norleucine, norvaline, sarcosine, homocitrulline, cysteic acid, τ-butylglycine, τ-butylalanine, phenylglycine, cyclohexylalanine, β-alanine, fluoro-amino acids, designer amino acids such as β-methyl amino acids, C-methyl amino acids, N-methyl amino acids, and amino acid analogues in general. Non-natural amino acids also include amino acids having derivatized side groups. Furthermore, any of the amino acids in the protein can be of the D (dextrorotary) form or L (levorotary) form.

Amino acids can be generally categorized in the following classes: non-polar, uncharged polar, basic, and acidic. Conservative substitutions whereby a modified protein of the present invention having an amino acid of one class is replaced with another amino acid of the same class fall within the scope of the subject invention so long as the modified protein having the substitution still retains substantially the same functional activity (e.g., increasing the levels of estradiol, estrogen, and/or progesterone) that does not have the substitution. Polynucleotides encoding a modified protein having one or more amino acid substitutions in the sequence are also within the scope of the present invention. Table 1 below provides a listing of examples of amino acids belonging to each class.

| **Table 1.** | |
|---|---|
| Class of Amino Acid | Examples of Amino Acids |
| Nonpolar | Ala, Val, Leu, Ile, Pro, Met, Phe, Trp |
| Uncharged Polar | Gly, Ser, Thr, Cys, Tyr, Asn, Gln |
| Acidic | Asp, Glu |
| Basic | Lys, Arg, His |

Polypeptides within the scope of the subject invention can also be defined in terms of more particular identity and/or similarity ranges with those sequences of the invention specifically exemplified herein. The sequence identity will typically be greater than 75%, preferably greater than 80%, more preferably greater than 90%, and can be greater than 95%. The identity and/or similarity of a sequence can be 75, 76, 77, 78, 79, 80, 81, 82, 83, 84, 85, 86, 87, 88, 89, 90, 91, 92, 93, 94, 95, 96, 97, 98, or 99% as compared to a sequence exemplified herein.

Unless otherwise specified, as used herein percent sequence identity and/or similarity of two sequences can be determined using the algorithm of Karlin and Altschul (1990), modified as in Karlin and Altschul (1993). Such an algorithm is incorporated into the NBLAST and XBLAST programs of Altschul *et al.* (1990). BLAST searches can be performed with the NBLAST program, score = 100, wordlength = 12, to obtain sequences with the desired percent sequence identity. To obtain gapped alignments for comparison purposes, Gapped BLAST can be used as described in Altschul *et al.* (1997). When utilizing BLAST and Gapped BLAST programs, the default parameters of the respective programs (NBLAST and XBLAST) can be used. *See* NCBI/NIH website.

Fragments of the polypeptides of the present invention, having activity including increasing the level estradiol, estrogen, and/or progesterone, can be generated using routine techniques. For example, a library of polypeptide fragments of various sizes can be generated from the full length DO peptide of the present invention; active fragments can be selected using assays taught in the present application, including testing the activity of the fragment in increasing the level of estradiol, estrogen, and/or progesterone; increasing the expression levels of aromatase CYP-19 and/or follicle-stimulating hormone receptor (FSHR); increasing the level of brain derived neurotrophic factor (BDNF) and/or TrkB receptor in hippocampus and prefrontal cortex; treating osteoporosis; e) improving cognitive function; and/or stimulating a splenic mitosis response.

The DO peptide of the present invention can conveniently be administered in a pharmaceutical composition containing the DO peptide at a clinically effectively concentration in combination with a suitable excipient formulation. The formulation may be selected to optimize delivery of DO peptide to the ovaries and thereby stimulate secretion of estrogen and progesterone. Such pharmaceutical compositions are prepared by methods and contain excipients that are well known in the art. A generally recognized compendium of such methods and ingredients is Remington's Pharmaceutical Sciences by E. W. Martin (Mark Publ. Co., 15.sup.th Ed., 1975). Compositions containing the DO peptide of the present invention can be administered parenterally, for example, by intravenous, intraperitoneal or intramuscular injection, topically, orally, or rectally.

Frequently, protein pharmaceuticals are rapidly eliminated from the systemic circulation by renal clearance and/or enzymatic degradation. They may also elicit an adverse immune response. As macromolecules, protein pharmaceuticals may not be readily taken up by their target cells, and may instead be taken up by non-target tissues or organs.

In one embodiment, renal filtration and clearance of the DO protein are addressed by conjugating the protein with one or more water-soluble polymers resulting in a complex larger than 40-kDa. Conjugation can also increase the resistance of the DO peptide to enzymatic degradation and decrease its immunogenicity. In one embodiment, the polymer is poly(ethylene glycol) (PEG).

In one embodiment, DO peptide is delivered using microreservoir carriers that provide a high degree of protection against enzymatic degradation by isolating the protein from the external environment. Examples of such microreservoirs include liposomes and nanoparticles.

Vector molecules with affinity towards characteristic ligands of target tissue can further improve the targeting of protein or peptide drug carriers. Vector molecules with this capability include antibodies, peptides, lectins, saccharides, hormones as well as small molecules such as vitamin. Among these molecules, antibodies have the highest potential specificity for targeting. In various embodiments, suitable vector molecules are used alone or in combination with protein conjugation, microreservoirs or other technologies to provide an optimal drug delivery system for DO peptide.

In one embodiment, the DO peptide is enterically coated, and delivered orally. For oral therapeutic administration, the active compound may be combined with one or more excipients and used in the form of ingestible tablets, buccal tablets, troches, capsules, elixirs, suspensions, syrups, wafers, and the like. Such compositions and preparations typically contain at least about 0.1% of active compound. The percentage of the compositions and preparations may, of course, be varied and may conveniently be between about 2 to about 60% of the weight of a given unit dosage form. The amount of active compound in such therapeutically useful compositions is such that an effective dosage level will be obtained.

The tablets, troches, pills, capsules, and the like may also contain the following: binders such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, fructose, lactose or aspartame or a flavoring agent such as peppermint, oil of wintergreen, or cherry flavoring may be added. When the unit dosage form is a capsule, it may contain, in addition to materials of the above type, a liquid carrier, such as a vegetable oil or a polyethylene glycol. Various other materials may be present as coatings or to otherwise modify the physical form of the solid unit dosage form. For instance, tablets, pills, or capsules may be coated with gelatin, wax, shellac or sugar and the like. A syrup or elixir may contain the active compound, sucrose or fructose as a sweetening agent, methyl and propylparabens as preservatives, a dye and flavoring such as cherry or orange flavor. Any material used in preparing any unit dosage form would be pharmaceutically acceptable and substantially non-toxic in the amounts employed. In addition, the DO peptide may be incorporated into sustained-release preparations and devices.

In one embodiment, pharmaceutical dosage forms suitable for injection or infusion of the DO peptide include sterile aqueous solutions or dispersions or sterile powders comprising the DO peptide which are adapted for the extemporaneous preparation of sterile injectable or infusible solutions or dispersions, optionally encapsulated in liposomes. In all cases, the ultimate dosage form should be sterile, fluid and stable under the conditions of manufacture and storage. The liquid carrier or vehicle can be a solvent or liquid dispersion medium comprising, for example, water, ethanol, a polyol (for example, glycerol, propylene glycol, liquid polyethylene glycols, and the like), vegetable oils, nontoxic glyceryl esters, and suitable mixtures thereof. The proper fluidity can be maintained, for example, by the formation of liposomes, by the maintenance of the required particle size in the case of dispersions or by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars, buffers or sodium chloride. Prolonged absorption of the injectable compositions can be brought about by the use in the compositions of agents delaying absorption, for example, aluminum monostearate and gelatin. Any of these injectable formulations may be suitable for use with the DosePro drug delivery system (Zogenix Corp., San Diego, CA, USA), or other similar devices.

Sterile injectable solutions can be prepared by incorporating the DO peptide in the required amount in the appropriate solvent with various of the other ingredients enumerated above, as required, followed by filter sterilization. In the case of sterile powders for the preparation of sterile injectable solutions, the preferred methods of preparation are vacuum drying and the freeze drying techniques, which yield a powder of the DO peptide plus any additional desired ingredient present in the previously sterile-filtered solutions.

For topical administration, the present compounds may be applied in pure form, i.e., when they are liquids. However, it will generally be desirable to administer them to the skin as compositions or formulations, in combination with a dermatologically acceptable carrier, which may be a solid or a liquid.

Useful solid carriers include finely divided solids such as talc, clay, microcrystalline cellulose, silica, alumina and the like. Useful liquid carriers include water, alcohols or glycols or water-alcohol/glycol blends, in which the DO peptide can be dissolved or dispersed at effective levels, optionally with the aid of non-toxic surfactants. Adjuvants such as fragrances and additional antimicrobial agents can be added to optimize the properties for a given use. The resultant liquid compositions can be applied from absorbent pads, used to impregnate bandages and other dressings, or sprayed onto the affected area using pump-type or aerosol sprayers. Thickeners such as synthetic polymers, fatty acids, fatty acid salts and esters, fatty alcohols, modified celluloses or modified mineral materials can also be employed with liquid carriers to form spreadable pastes, gels, ointments, soaps, and the like, for application directly to the skin of the user.

Useful dosages of the DO peptide of the invention can be determined from it apparent in vivo activity in animal models. Methods for the extrapolation of effective dosages in rats and other animals, to humans are known to the art. In the present invention, the effective dose in aged Sprague-Dawley rats is in the range of 2.5 to 10.0 mg/kg. In one embodiment the effective dose for human patients is in the range of 0.5 to 500 mg/kg.

In one embodiment, the DO peptide is conveniently administered in unit dosage form; for example, containing 1 to 10 g, of DO peptide per unit dosage form. The desired dose may conveniently be presented in a single dose or as divided doses administered at appropriate intervals, for example, as two, three, four or more sub-doses per day.

The exact regimen for administration of the DO peptide can be obtained depending upon the needs of the individual patient being treated, the type of treatment and, of course, the judgment of the attending practitioner.

In one embodiment the DO peptide is extracted for the rhizomes of the Chinese yam *Discorea Opposita,* as described above. In another embodiment the DO peptide is produced by recombinant means.
In one aspect of the present invention a therapeutic composition, comprising an isolated or substantially pure polypeptide compound of the present invention, or a salt thereof, and a pharmaceutically acceptable carrier is provided.

The term "carrier" refers to a diluent, adjuvant, excipient, or vehicle with which the compound is administered. Such pharmaceutical carriers can be sterile liquids, such as water and oils, including those of petroleum oil such as mineral oil; vegetable oil such as peanut oil, soybean oil, and sesame oil; animal oil; or oil of synthetic origin. Saline solutions and aqueous dextrose and glycerol solutions can also be employed as liquid carriers, particularly for injectable solutions.

The therapeutic or pharmaceutical compositions of the invention can be formulated as neutral or salt forms. Pharmaceutically acceptable salts include, but are not limited to, salts formed with hydrochloric, phosphoric, acetic, oxalic, tartaric acids, sodium, potassium, ammonium, calcium, ferric hydroxides, etc.

The invention also provides a pharmaceutical pack or kit comprising one or more containers filled with one or more of the ingredients, e.g., compound, carrier, of the pharmaceutical compositions of the invention.

The compounds and compositions of the subject invention can be administered to the subject being treated by standard routes, including oral, inhalation, or parenteral administration including intravenous, subcutaneous, topical, transdermal, intradermal, transmucosal, intraperitoneal, intramuscular, intracapsular, intraorbital, intracardiac, transtracheal, subcutaneous, subcuticular, intraarticular, subcapsular, subarachnoid, intraspinal, epidural and intrasternal injection, infusion, and electroporation, as well as co-administration as a component of any medical device or object to be inserted (temporarily or permanently) into a subject. In preferred embodiments, the compounds and compositions of the subject invention are administered to a subject by oral administration.

The amount of the therapeutic or pharmaceutical composition which is effective in the treatment of a particular disease, condition or disorder will depend on the route of administration, and the seriousness of the disease, condition or disorder, and should be decided according to the judgment of the practitioner and each patient's circumstances. In general, the dosage ranges from about 0.001 mg/kg to about 3 g/kg.

The amount of active ingredient that may be combined with the carrier materials to produce a single dosage form will vary, depending on the type of the condition and the subject to be treated. In general, a therapeutic composition contains from about 5% to about 95% active ingredient (w/w). More specifically, a therapeutic composition contains from about 20% (w/w) to about 80% or about 30% to about 70% active ingredient (w/w).

Once improvement of the patient's condition has occurred, a maintenance dose is administered if necessary. Subsequently, the dosage or the frequency of administration, or both, may be reduced as a function of the symptoms to a level at which the improved condition is retained. When the symptoms have been alleviated to the desired level, treatment should cease. Patients may however require intermittent treatment on a long-term basis upon any recurrence of disease symptoms.

## Claims

1. A peptide that increases the level of estrogen in vitro or in vivo, wherein the peptide is an isolated or substantially pure polypeptide obtainable from Dioscorea sp., wherein the polypeptide has an apparent molecular weight of about 32.5 kDA by chromatography, wherein the first twenty-one consecutive amino acids at the N-terminal of the polypeptide consist of SEQ ID NO:1.

2. A peptide according to claim 1, wherein the peptide increases the level of progesterone in vitro or in vivo.

3. A peptide according to claim 1, which is an isolated or substantially pure polypeptide obtainable from Dioscorea sp., wherein the polypeptide has an apparent molecular weight of about 32.5 kDA by chromatography, and wherein the first twenty-one consecutive amino acids at the N-terminal of the polypeptide consists of SEQ ID NO:1.

4. A peptide according to claim 1, wherein the polypeptide originates from Discorea Opposita.

5. A peptide according to claim 1, which is recombinantly-produced.

6. A peptide according to claim 1, wherein the peptide has higher activity after incubation at pH 1 than after incubation at pH 0 or at pH 2, wherein the activity is increasing the level of estrogen and/or progesterone in an ovarian cell or in a female subject.

7. A peptide according to claim 6, wherein the peptide has activity after incubation at pH 0, wherein the activity is increasing the level of estrogen and/or progesterone in an ovarian cell or in a female subject.

8. A peptide according to claim 1, wherein the peptide has higher activity after incubation at 80°C than after incubation at 60°C or at 100°C, wherein the activity is increasing the level of estrogen and/or progesterone in an ovarian cell or in a female subject.

9. A peptide according to claim 8, wherein the peptide has activity after incubation at 100°C, wherein the activity is increasing the level of estrogen and/or progesterone in an ovarian cell or in a female subject.

10. An antibody that binds specifically to a peptide according to claim 1.

## Patentansprüche

1. Peptid, welches den Östrogenspiegel in vitro oder in vivo erhöht, wobei das Peptid ein isoliertes oder im Wesentlichen reines Polypeptid ist, das aus Dioscorea sp. erhältlich ist, wobei das Polypeptid ein scheinbares Molekulargewicht von etwa 32,5 kDa gemäß Chromatographie aufweist, wobei die ersten einundzwanzig aufeinander folgenden Aminosäuren am N-Terminus des Polypeptids aus SEQ ID NO:1 bestehen.

2. Peptid nach Anspruch 1, wobei das Peptid den Progresteronspiegel in vitro oder in vivo erhöht.

3. Peptid nach Anspruch 1, das ein isoliertes oder im Wesentlichen reines Polypeptid ist, das aus Dioscorea sp. erhältlich ist, wobei das Polypeptid ein scheinbares Molekulargewicht von etwa 32,5 kDa gemäß Chromatographie aufweist, und wobei die ersten einundzwanzig aufeinander folgenden Aminosäuren am N-Terminus des Polypeptids aus SEQ ID NO:1 bestehen.

4. Peptid nach Anspruch 1, wobei das Polypeptid seinen Ursprung in Discorea Opposita hat.

5. Peptid nach Anspruch 1, das rekombinant produziert ist.

6. Peptid nach Anspruch 1, wobei das Peptid eine höhere Aktivität nach Inkubation bei pH 1 als nach Inkubation bei pH 0 oder pH 2 aufweist, wobei die Aktivität den Spiegel von Östrogen und/oder Progesteron in einer Ovarialzelle oder einem weiblichen Subjekt erhöht.

7. Peptid nach Anspruch 6, wobei das Peptid Aktivität nach Inkubation bei pH 0 aufweist, wobei die Aktivität den Spiegel von Östrogen und/oder Progesteron in einer Ovarialzelle oder einem weiblichen Subjekt erhöht.

8. Peptid nach Anspruch 1, wobei das Peptid eine höhere Aktivität nach Inkubation bei 80 °C als nach Inkubation bei 60 °C oder bei 100 °C aufweist, wobei die Aktivität den Spiegel von Östrogen und/oder Progesteron in einer Ovarialzelle oder einem weiblichen Subjekt erhöht.

9. Peptid nach Anspruch 8, wobei das Peptid Aktivität nach Inkubation bei 100 °C aufweist, wobei die Aktivität den Spiegel von Östrogen und/oder Progesteron in einer Ovarialzelle oder einem weiblichen Subjekt erhöht.

10. Antikörper, der spezifisch an ein Peptid gemäß Anspruch 1 bindet.

## Revendications

1. Peptide qui augmente le taux d'oestrogène in vitro ou in vivo, le peptide étant un polypeptide isolé ou sensiblement pur pouvant être obtenu à partir de Dioscorea sp., le polypeptide ayant une masse moléculaire apparente d'environ 32,5 kDA par chromatographie, les premiers vingt-et-un acides aminés consécutifs au niveau de l'extrémité N-terminale du polypeptide consistant en la séquence SEQ ID NO : 1.

2. Peptide selon la revendication 1, le peptide augmentant le taux de progestérone in vitro ou in vivo.

3. Peptide selon la revendication 1, qui est un polypeptide isolé ou sensiblement pur pouvant être obtenu à partir de Dioscorea sp., le polypeptide ayant une masse moléculaire apparente d'environ 32,5 kDA par chromatographie, et les premiers vingt-et-un acides aminés consécutifs au niveau de l'extrémité N-terminale du polypeptide consistant en la séquence SEQ ID NO : 1.

4. Peptide selon la revendication 1, le polypeptide provenant de Discorea Opposita.

5. Peptide selon la revendication 1, qui est obtenu par recombinaison.

6. Peptide selon la revendication 1, le peptide ayant une activité plus élevée après incubation à pH 1 qu'après une incubation à pH 0 ou à pH 2, l'activité augmentant le taux d'oestrogène et/ou de progestérone dans une cellule ovarienne ou chez un sujet féminin.

7. Peptide selon la revendication 6, le peptide ayant une activité après incubation à pH 0, l'activité augmentant le taux d'oestrogène et/ou de progestérone dans une cellule ovarienne ou chez un sujet féminin.

8. Peptide selon la revendication 1, le peptide ayant une activité plus élevée après incubation à 80 °C qu'après incubation à 60 °C ou à 100 °C, l'activité augmentant le taux d'oestrogène et/ou de progestérone dans une cellule ovarienne ou chez un sujet féminin.

9. Peptide selon la revendication 8, le peptide ayant une activité après incubation à 100 °C, l'activité augmentant le taux d'oestrogène et/ou de progestérone dans une cellule ovarienne ou chez un sujet féminin.

10. Anticorps qui se lie spécifiquement à un peptide selon la revendication 1.
